(19) **Europäisches Patentamt / European Patent Office / Office européen des brevets**

(11) **EP 4 059 518 A1**

(12) **EUROPEAN PATENT APPLICATION**
published in accordance with Art. 153(4) EPC

(43) Date of publication:
**21.09.2022 Bulletin 2022/38**

(21) Application number: **20888498.1**

(22) Date of filing: **04.11.2020**

(51) International Patent Classification (IPC):
**A61K 39/395** (2006.01)     **A61K 45/00** (2006.01)
**A61K 45/06** (2006.01)     **A61P 31/00** (2006.01)
**A61P 35/00** (2006.01)     **A61P 43/00** (2006.01)
**A61K 31/132** (2006.01)

(52) Cooperative Patent Classification (CPC):
**A61K 31/132; A61K 39/395; A61K 45/00;
A61K 45/06; A61P 31/00; A61P 35/00; A61P 43/00**

(86) International application number:
**PCT/JP2020/041190**

(87) International publication number:
**WO 2021/095599 (20.05.2021 Gazette 2021/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.11.2019  JP 2019205550**

(71) Applicant: **Kyoto University
Kyoto-shi, Kyoto 606-8501 (JP)**

(72) Inventors:
• **HONJO, Tasuku**
  **Kyoto-shi, Kyoto 606-8501 (JP)**
• **CHAMOTO, Kenji**
  **Kyoto-shi, Kyoto 606-8501 (JP)**
• **FAGARASAN, Sidonia**
  **Wako-shi, Saitama 351-0198 (JP)**

(74) Representative: **J A Kemp LLP
80 Turnmill Street
London EC1M 5QU (GB)**

(54) **PD-1 SIGNAL INHIBITOR COMBINATION THERAPY**

(57)    The present invention provides a novel therapeutic strategy for anti-PD-1 antibody therapy. The present invention relates to a pharmaceutical composition which increases the function of oxidative phosphorylation in T cells; and a pharmaceutical composition which has an action for increasing the function of oxidative phosphorylation in T cells and is administered before, after or simultaneously with the administration of a PD-1 signaling inhibitor.

EP 4 059 518 A1

**Figure 1-1**

a)

levels in cells

levels in supernatant

b)

- ○ CTRL
- ▲ PD-L1 mAb
- ● PD-L1 mAb+ 2mg/kg SPD (2/5 cured)
- △ SPD 1

Days after MC38 inoculation

Days after MC38 inoculation

**Figure 1-1 cont.**

c) Spermidine increases the frequency and number of effector phenotype CD8 T cells in DLN

**(Cont. next page)**

**Figure 1-1 cont.**

d) Spermidine increases CD8[*] T cells infiltrating tumor mass when combined with anti-PD-L1 monoclonal antibodies

**Description**

Technical Field

The present invention relates to a combination therapy using a PD-1 signaling inhibitor.

Background Art

The results of recent clinical trials have revealed that the anti-PD-1 antibody therapy is more effective than conventional standard therapies in various cancers (Non-Patent Documents Nos. 1-3). The response rate of PD-1 antibody therapy in terminal lung cancer patients was 20-30%, showing a dramatic improvement compared to conventional anti-cancer drug therapies. However, about one half of the patients were non-responsive. Little is known about why those patients are non-responsive to the PD-1 antibody therapy.

Prior Art Literature

Non-Patent Documents

Non-Patent Document No. 1: Bramer J, Reckamp K, et al: Nivolumab versus Docetaxel in Advanced Nonsquamous Non-Small-Cell Lung Cancer. N Engl J Med, 373:1627-1639, 2015
Non-Patent Document No. 2: Hamanishi J, Mandai M, Ikeda T, et al: Safety and Antitumor Activity of Anti-PD-1 Antibody, Nivolumab, in Patients with Platinum-Resistant Ovarian Cancer. J Clin Oncol, 33:4015-4022, 2015
Non-Patent Document No. 3: Motzer RJ, Escudier B, McDermott DF, et al: Nivolumab versus Everolimus in Advanced Renal-Cell Carcinoma. N Engl J Med, 373:1803-1813, 2015

Summary of the Invention

Problem for Solution by the Invention

It is an object of the present invention to provide a novel therapeutic strategy for anti-PD-1 antibody therapy.

Means to Solve the Problem

Unlike the conventional anti-cancer drugs which have a direct killing effect on cancer cells, anti-PD-1 antibody therapy inhibits cancer proliferation by activating antitumor immunity. It is expected that antitumor effect can be improved by a combined use of anti-PD-1 antibody and reagents which support antitumor immunity. Such a combination therapy may be applicable to non-responsive patients.

The present inventors have found that antitumor effect is synergistically improved when PD-1 signaling inhibiting antibodies are used in combination with spermidine (a kind of polyamine). Spermidine alone exhibited no antitumor effect in living bodies. Further, spermidine increased the function of oxidative phosphorylation in T cells. From these results, it is considered that substances typified by spermidine which increase the function of oxidative phosphorylation in T cells support antitumor immunity and synergistically inhibit cancer proliferation when used in combination with PD-1 signaling inhibiting antibody.

A summary of the present invention is as described below.

(1) A pharmaceutical composition which increases the function of oxidative phosphorylation in T cells.
(2) The pharmaceutical composition of (1) above, which comprises a polyamine.
(3) The pharmaceutical composition of (1) or (2) above, wherein the polyamine comprises at least one member selected from the group consisting of spermine, spermidine, putrescine, salts thereof and solvates thereof.
(4) A pharmaceutical composition which has an action for increasing the function of oxidative phosphorylation in T cells and is administered before, after or simultaneously with the administration of a PD-1 signaling inhibitor.
(5) The pharmaceutical composition of (4) above, which comprises a polyamine.
(6) The pharmaceutical composition of (5) above, wherein the polyamine comprises at least one member selected from the group consisting of spermine, spermidine, putrescine, salts thereof and solvates thereof.
(7) The pharmaceutical composition of any one of (4) to (6) above, wherein the PD-1 signaling inhibitor is an antibody.
(8) The pharmaceutical composition of (7) above, wherein the antibody is at least one antibody selected from the group consisting of anti-PD-1 antibody, anti-PD-Ll antibody and anti-PD-L2 antibody.

(9) The pharmaceutical composition of any one of (4) to (8) above, which is used as an anti-cancer agent, an anti-infective agent or a combination thereof.

(10) The pharmaceutical composition of any one of (4) to (9) above, wherein the PD-1 signaling inhibitor and the pharmaceutical composition are administered separately.

(11) The pharmaceutical composition of any one of (4) to (9) above, which is a combination drug comprising the PD-1 signaling inhibitor and the pharmaceutical composition.

(12) A method of treating cancer, infection or a combination thereof, comprising administering to a human or animal subject a pharmaceutically effective amount of a pharmaceutical composition which increases the function of oxidative phosphorylation in T cells, wherein the composition is administered to the subject before, after or simultaneously with the administration of a PD-1 signaling inhibitor.

(13) Use of a pharmaceutical composition which increases the function of oxidative phosphorylation in T cells for treating cancer, infection or a combination thereof, wherein the composition is administered before, after or simultaneously with the administration of a PD-1 signaling inhibitor.

(14) Use of a pharmaceutical composition which increases the function of oxidative phosphorylation in T cells for a method for treating cancer, infection or a combination thereof, wherein the composition is administered before, after or simultaneously with the administration of a PD-1 signaling inhibitor.

Effect of the Invention

Antitumor effect is synergistically improved if substances that increase the function of oxidative phosphorylation in T cells are used in combination with PD-1 signaling inhibitors.

The present specification encompasses the contents disclosed in the specification and/or drawings of Japanese Patent Application No. 2019-205550 based on which the present application claims priority.

Brief Description of the Drawings

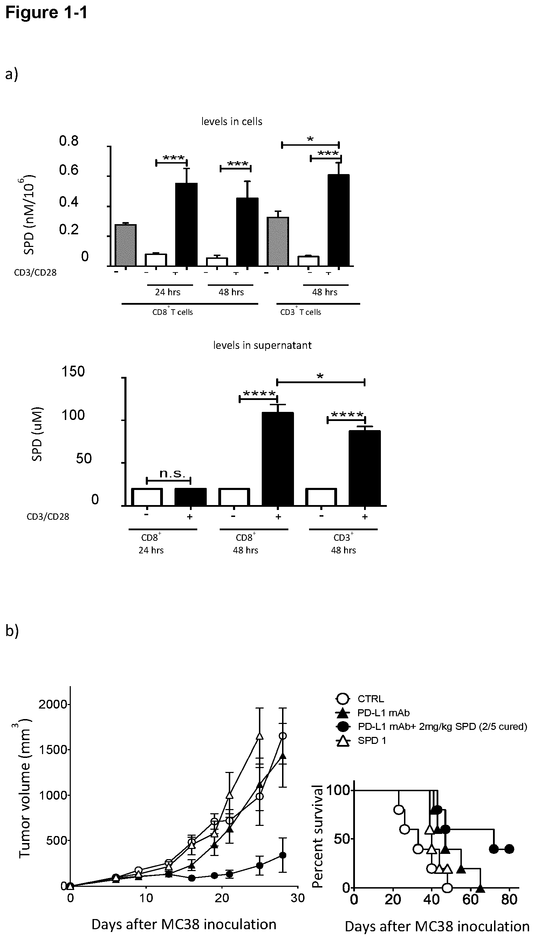

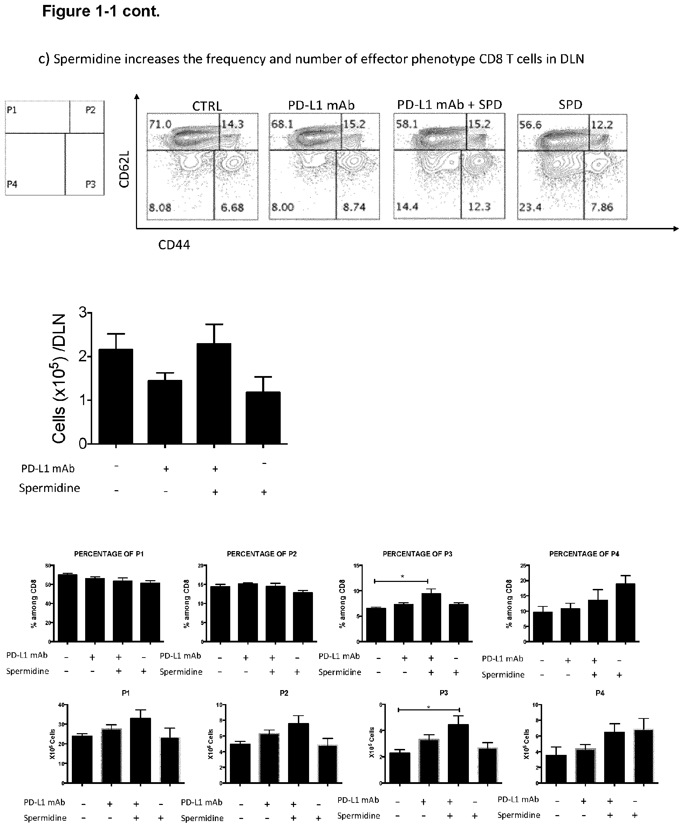

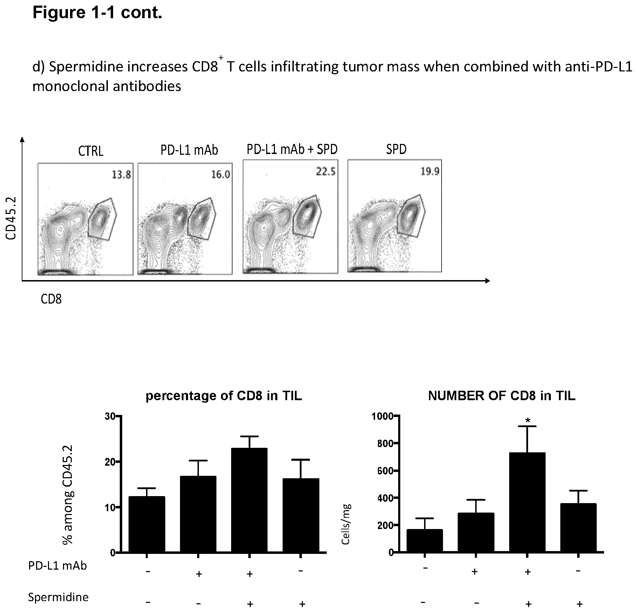

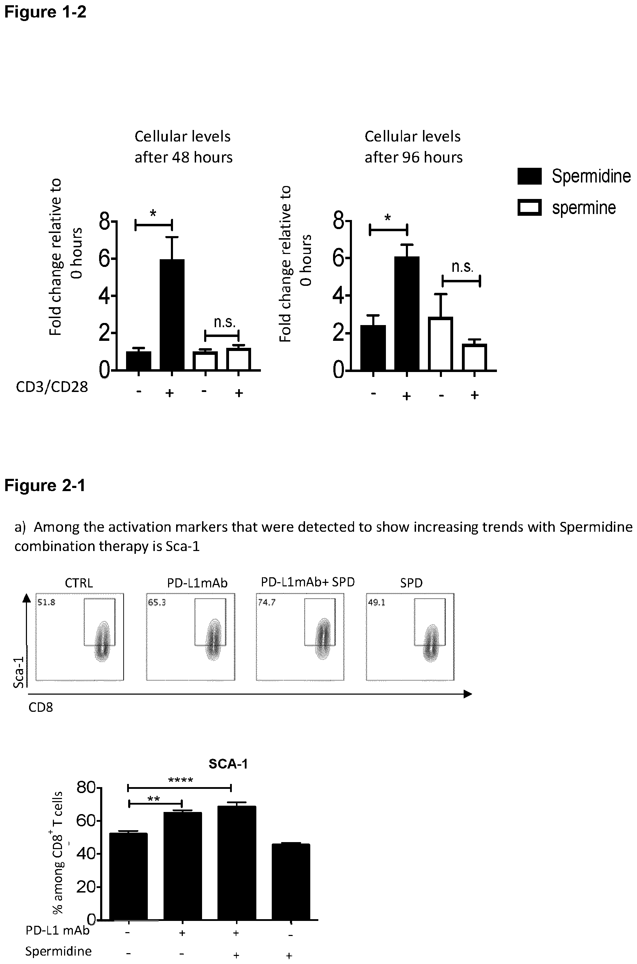

[Fig. 1-1] a) CD8$^+$ T cells or CD3$^+$ T cells were isolated from mice, and $2 \times 10^5$ cells were stimulated with CD3/CD28 beads in 96-well U plates. After 24 hours and 48 hours, cells and culture supernatant were collected, followed by measurement of spermidine (SPD) concentration by mass spectrometry. The upper graph shows SPD levels in the cells and the lower graph, SPD levels in the supernatant. b) MC38 cells ($5 \times 10^5$) were intradermally inoculated into C57BL/6 mice. Ten days after inoculation, PD-L1 antibody (2 mg/kg), PD-L1 antibody + SPD (2 mg/kg), or SPD alone was administered. Subsequently, PD-L1 antibody was administered 4 times at a regular interval of 5 days, and SPD was administered 8 times at a regular interval of 3 days. Tumor growth curve and survival curve are shown. c) Draining lymph nodes (DLNs) of mice were isolated 13 days after tumor inoculation, and stained with antibodies to CD8, CD44 and CD62L. The number of cells in draining lymph node (cells/DLN) in each treatment group is shown at the left side. After being gated, CD8$^+$ T cells were fractionated with CD44 and CD62L into P1 to P4 cell populations. Percentages and cell numbers of P1 to P4 in each treatment group are shown. d) Tumor was isolated 13 days after tumor inoculation and treated with collagenase. Percentages and numbers per mg tumor of tumor-infiltrating CD8$^+$ CD45.2$^+$ cells are shown. For statistical analysis, ANOVA was used. * $p<0.05$, ** $p<0.01$, *** $p<0.001$, **** $p<0.0001$.

[Fig. 1-2] CD3$^+$ T cells were isolated from mice, and $2x10^5$ cells were stimulated with CD3/CD28 beads in 96-well U plates. After 48 hours and 96 hours, cells were collected, followed by measurement of SPD and spermine (SPM) concentrations by mass spectrometry. Taking the cellular level before stimulation as 1, fold change is shown.

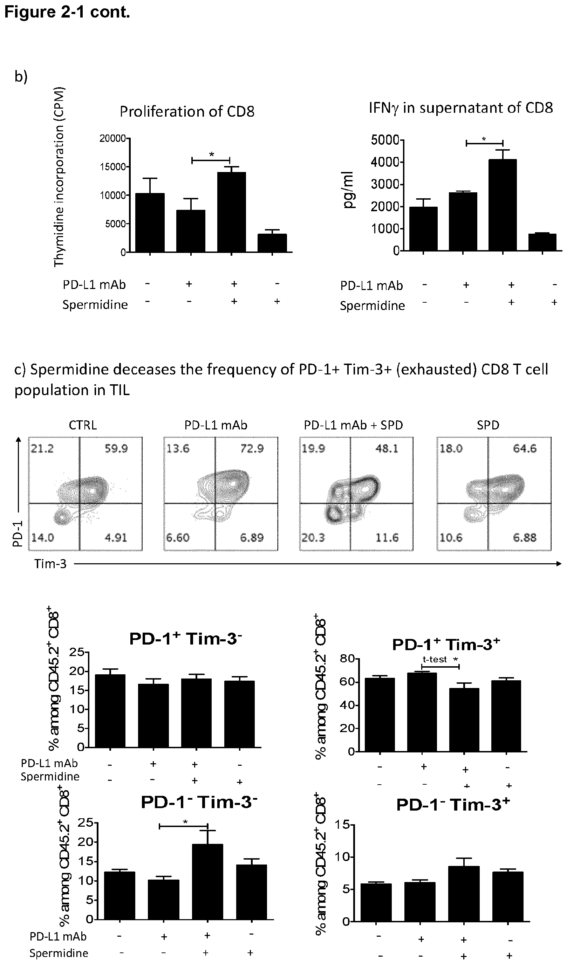

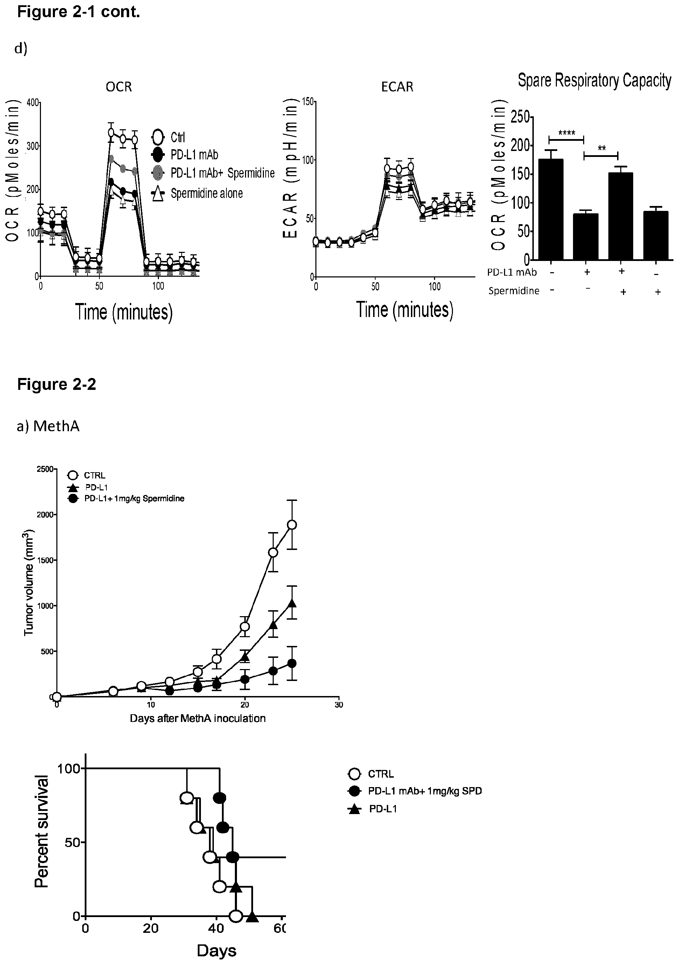

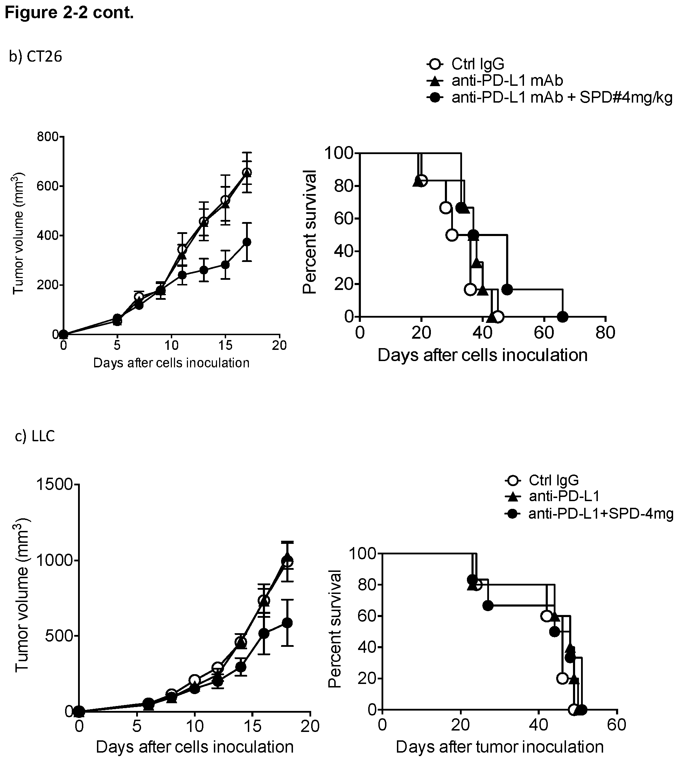

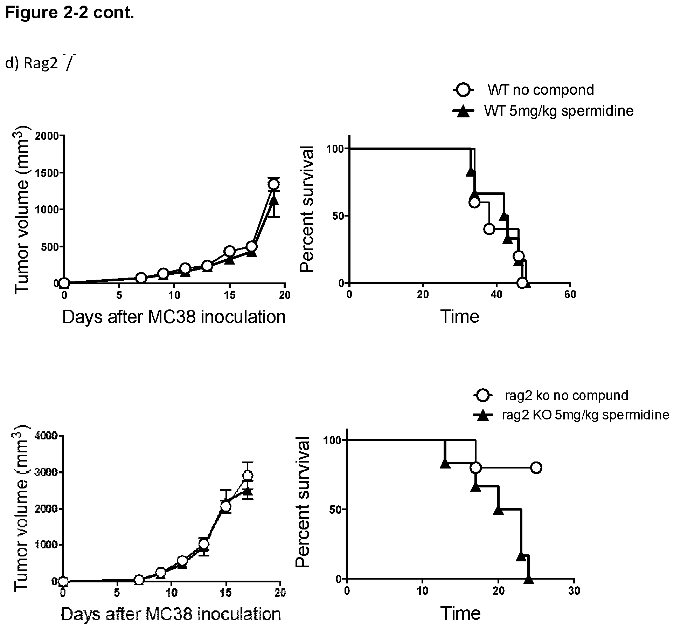

[Fig. 2-1] a) Treated mice were analyzed for draining lymph nodes and the inside of tumor. Briefly, draining lymph nodes of mice were isolated 13 days after tumor inoculation, and stained with antibodies to CD8 and Sca-1. FACS diagram and percentages of Sca-1$^+$ cells in CD8$^+$ T cells are shown. b) CD8$^+$ T cells in isolated draining lymph nodes were stimulated with CD3/CD28 antibody beads. After 24 hours, cell proliferation was examined by means of $^3$H uptake. At the same time, IFN-$\gamma$ concentration in culture supernatant was measured. c) Tumor from the same mice as described above was treated with collagenase. Then, expression rates of PD-1 and Tim3 in tumor-infiltrating CD8$^+$ T cells were analyzed. d) Oxygen consumption rate (OCR) and extracellular acidification rate (ECAR) of draining lymph node CD8$^+$T cells were measured with a Seahorse analyzer (N=3 wells). For statistical analysis, ANOVA was used. * $p<0.05$, ** $p<0.01$, *** $p<0.001$, **** $p<0.0001$.

[Fig. 2-2] a-b) MethA or CT26 cells were intradermally inoculated into BALB/c mice. Ten days after inoculation, PD-L1 antibody (2 mg/kg), PD-L1 antibody + SPD (1 mg/kg for Meth A, 4 mg/kg for CT26) or SPD alone was administered. c) LLC cells were intradermally inoculated into C57BL/6 mice. Ten days after inoculation, mice were treated in the same manner as described in a-b) above. SPD was administered at 4 mg/kg. d) MC38 cells were inoculated into C57BL/6 Rag2$^{-/-}$ mice. Ten days after inoculation, mice were treated in the same manner as described in a-b) above. SPD was administered at 5 mg/kg. Tumor growth curve and survival curve are shown.

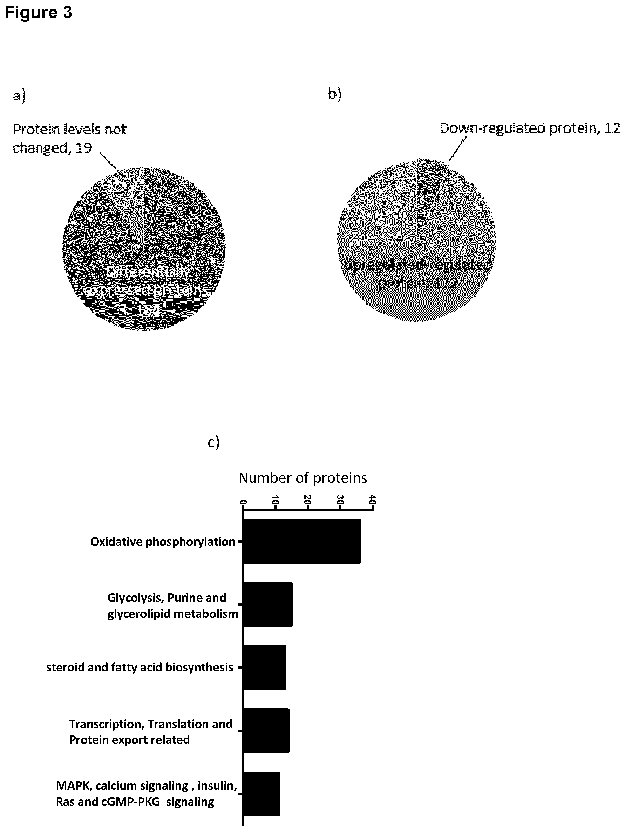

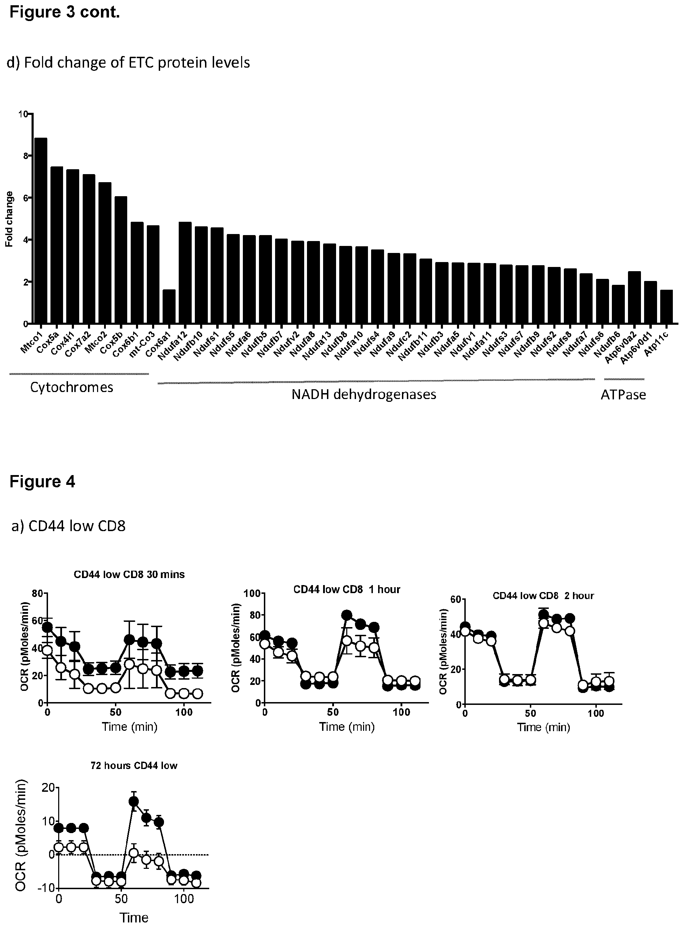

[Fig. 3] Draining lymph node CD8$^+$ T cells were isolated 13 days after tumor inoculation, and mitochondria-related proteomics analyses were conducted. a) The number of mitochondria-related proteins whose expression level changed significantly in the group treated with PD-1 antibody + SPD compared to the group treated with PD-1 antibody alone; and b) the number of proteins whose expression level significantly increased or decreased are shown. c) Pathway analysis (GO analysis) was conducted using 184 proteins whose expression level increased significantly in the group treated with PD-1 antibody + SPD compared to the group treated with PD-1 antibody alone. Pathway categories showing significant increase and the number of the associated proteins are shown. d) Among the proteins which significantly increased, fold change of expression levels of the proteins related to an electron chain (ETC) is shown.

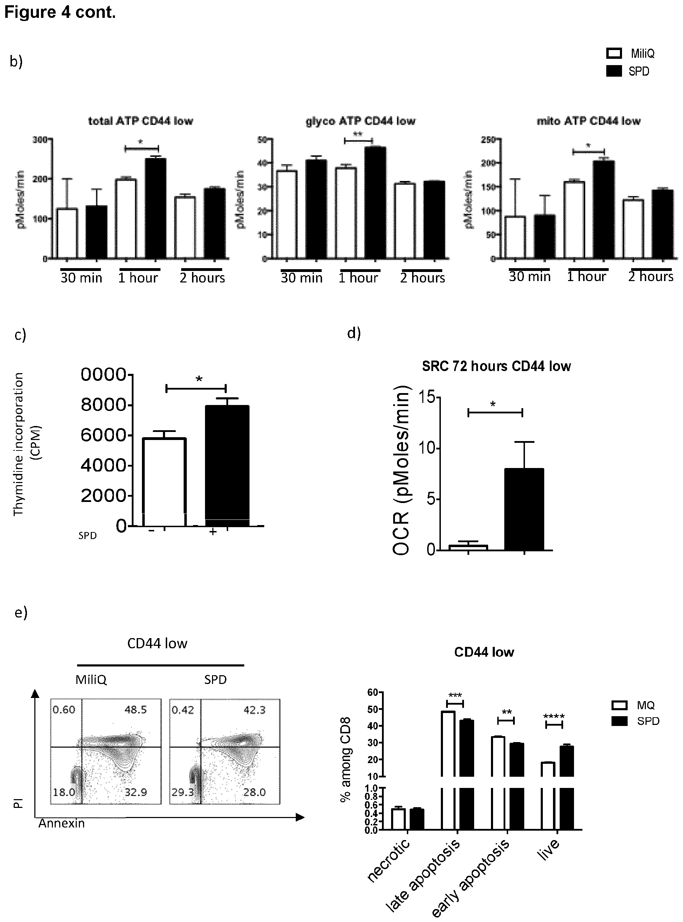

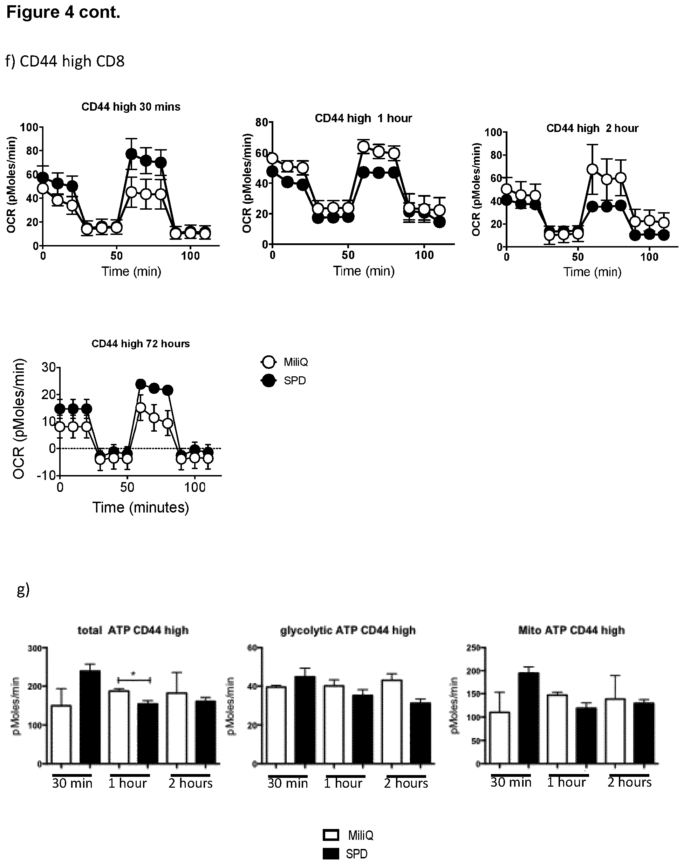

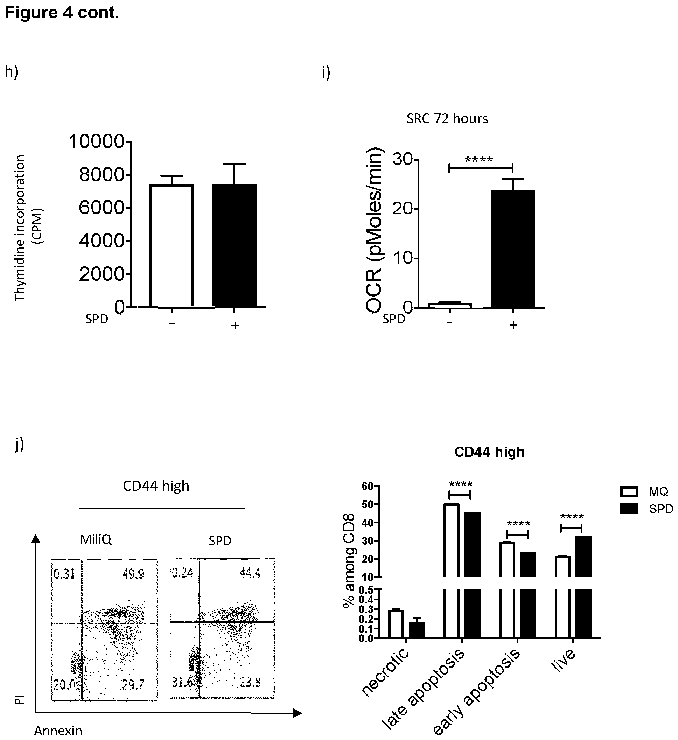

[Fig. 4] CD8$^+$ T cells were isolated from the spleen of mice without tumor inoculation, and further isolated into CD44$^+$ (CD44 high) and CD44$^-$ (CD44 low) cell populations. a) CD44 low cells were simulated with CD3/CD28 antibody beads and SPD (0.2 $\mu$M). After 30 minutes, 1 hour, 2 hours and 72 hours, OCR was measured with a Seahorse analyzer. b) At the same time, total ATP production and glycolytic pathway-derived or mitochondrial activity-derived ATP production were calculated at 30-minute, 1-hour and 2-hour. c) CD44 low cells (2x10$^5$ cells) were stimulated with CD3/CD28 antibody beads and SPD (0.2 $\mu$M). After 24 hours, cell proliferation was examined by means of $^3$H uptake. d) Spare respiratory capacity (SRC) was calculated from the OCR measurement after 72 hours in a), and results with and without SPD were compared. e) CD44 low cells were stimulated with CD3/CD28 antibody beads and SPD (0.2 $\mu$M). After 24 hours, frequencies between apoptotic and viable cells were calculated by PI and Annexin staining. f-j) CD44 high cells were analyzed under the same conditions and by the same methods as described in a-e) above. As regards statistical analysis, t-test was used for comparison of two groups, and ANOVA was used for comparison of three or more groups. * $p<0.05$, ** $p<0.01$, *** $p<0.001$, **** $p<0.0001$.

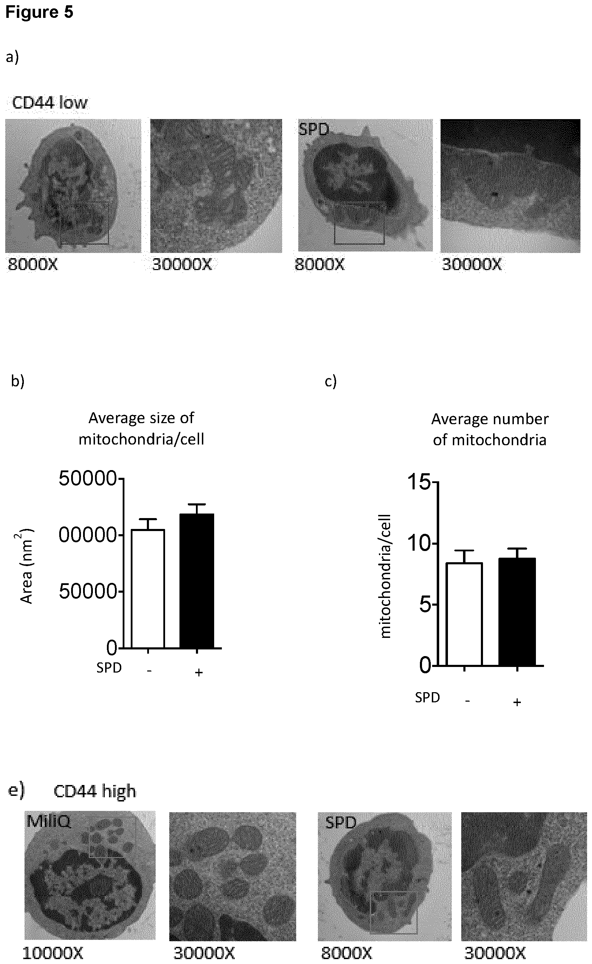

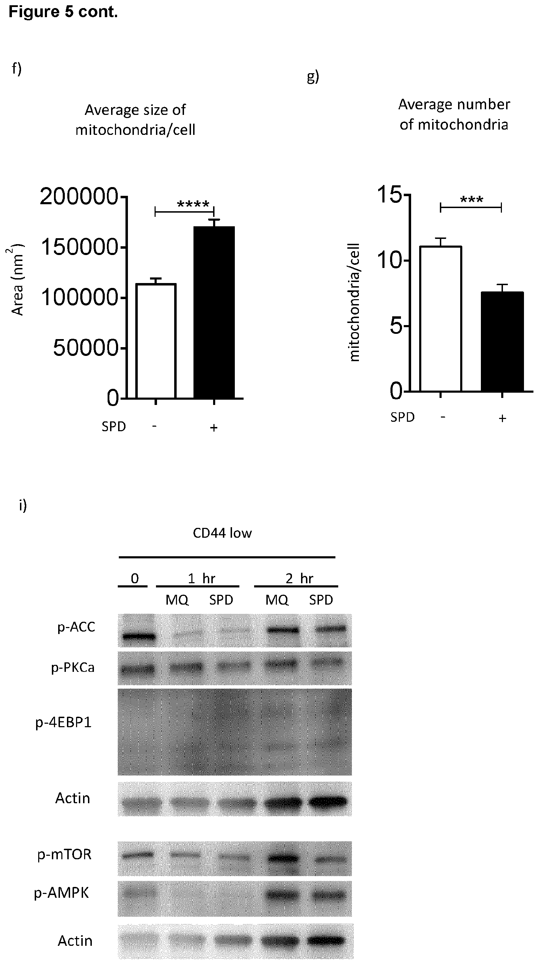

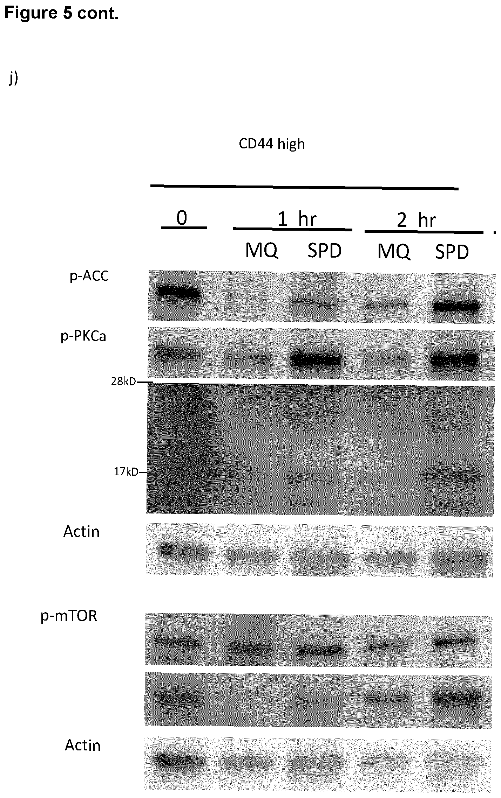

[Fig. 5] CD8$^+$ T cells were isolated from the spleen of mice without tumor inoculation, and further isolated into CD44$^+$ (CD44 high) and CD44$^-$ (CD44 low) cell populations. a) CD44 low cells were simulated with CD3/CD28 antibody beads and SPD (0.2 $\mu$M). After 1 hour, mitochondrial morphology was observed with an electron microscope. b) The size of mitochondria (mm$^2$) per cell was calculated with ImageJ. c) The number of mitochondria per cell was calculated. e-g) CD44$^+$ (CD44 high) cell populations were analyzed under the same conditions and by the same methods as described in a-c) above. i-j) CD44 low cells (i) and CD44 high cells (j) were stimulated with CD3/CD28 antibody beads and ultra-pure water (MQ) or SPD (0.2 $\mu$M). Protein expression levels after 1 hour and 2 hours were confirmed by western blotting with the respective indicated antibodies. As regards statistical analysis, t-test was used for comparison of two groups. * $p<0.05$, ** $p<0.01$, *** $p<0.001$, **** $p<0.0001$.

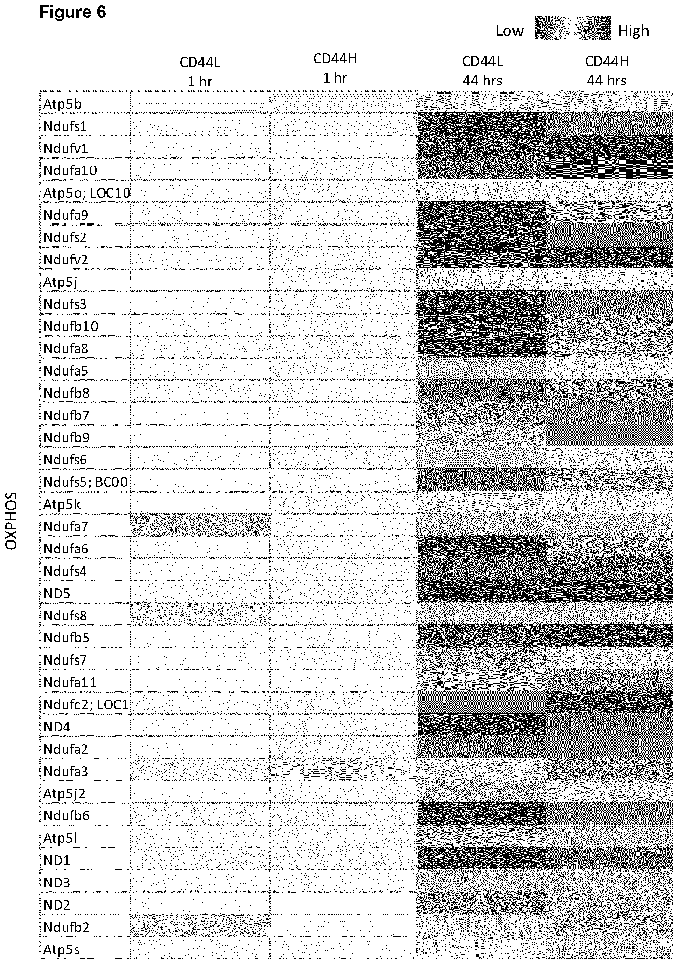

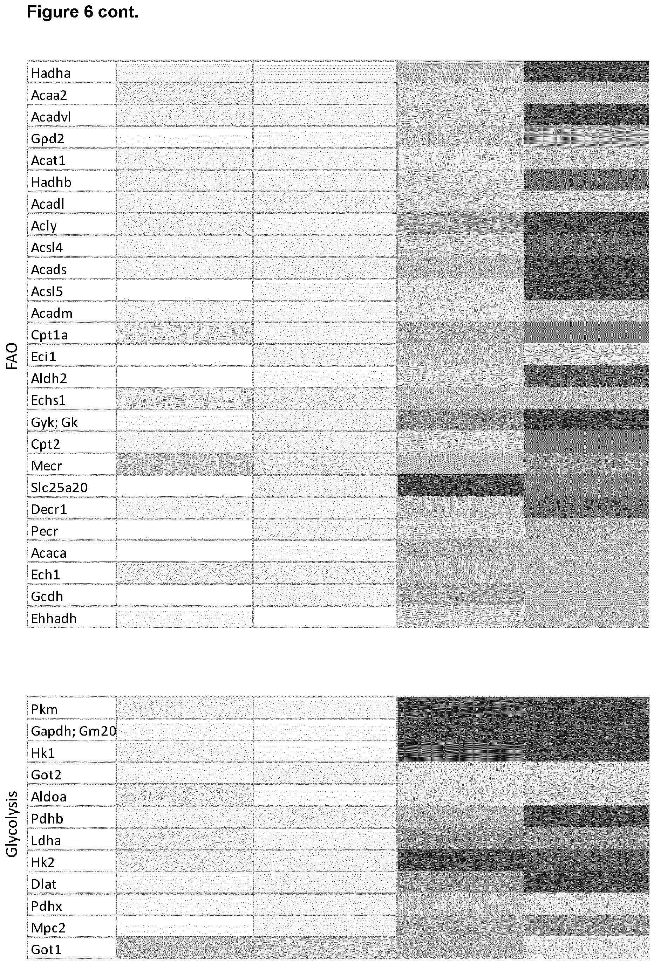

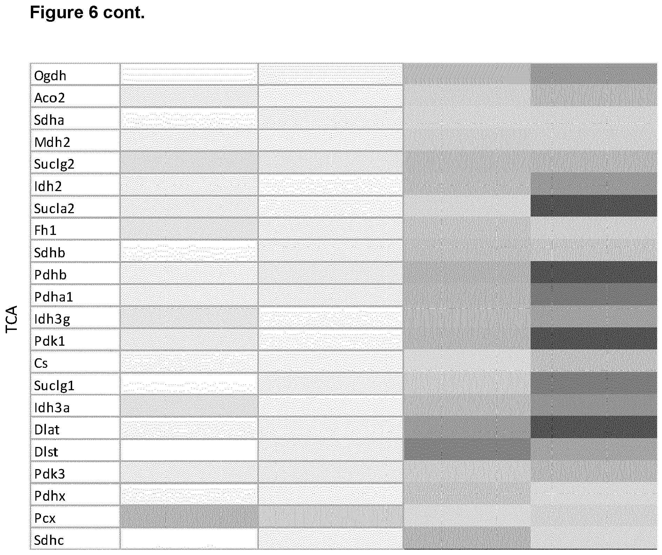

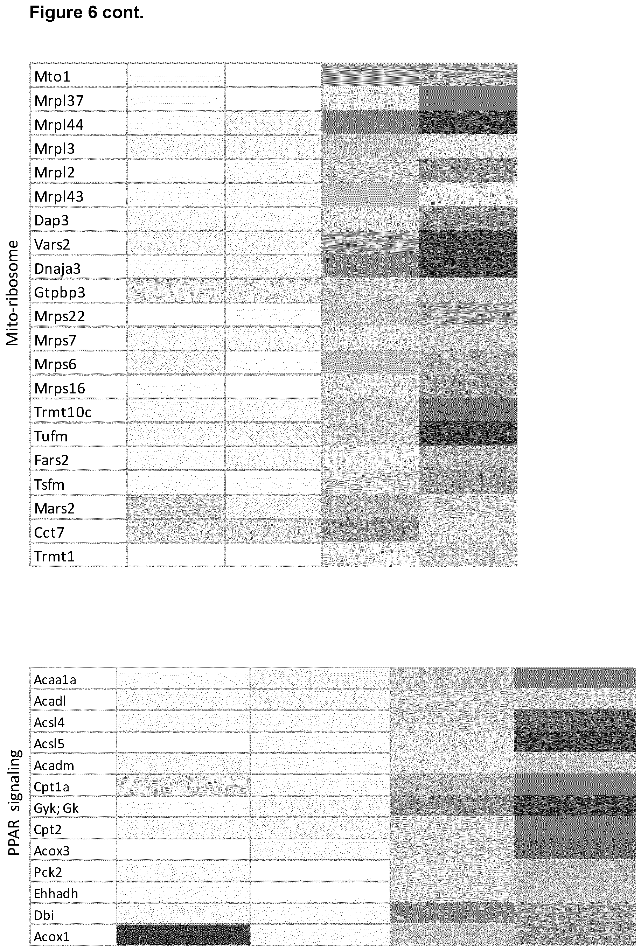

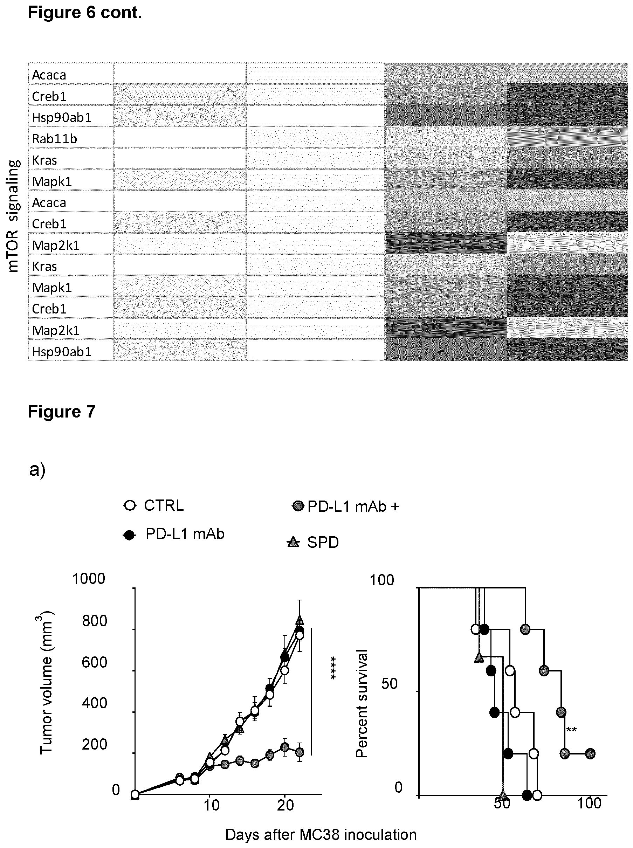

[Fig. 6] CD8$^+$ T cells were isolated from the spleen of mice without tumor inoculation, and further isolated into CD44$^+$ (CD44 high) and CD44$^-$ (CD44 low) cell populations. CD44 low cells and CD44 high cells were simulated with CD3/CD28 antibody beads and SPD (0.2 $\mu$M). After 1 hour and 44 hours, cells were collected and proteomics analyses regarding mitochondria-related proteins were conducted. A heat map showing the categories of respective signaling pathways, associated protein names and comparison of their expression levels is given.

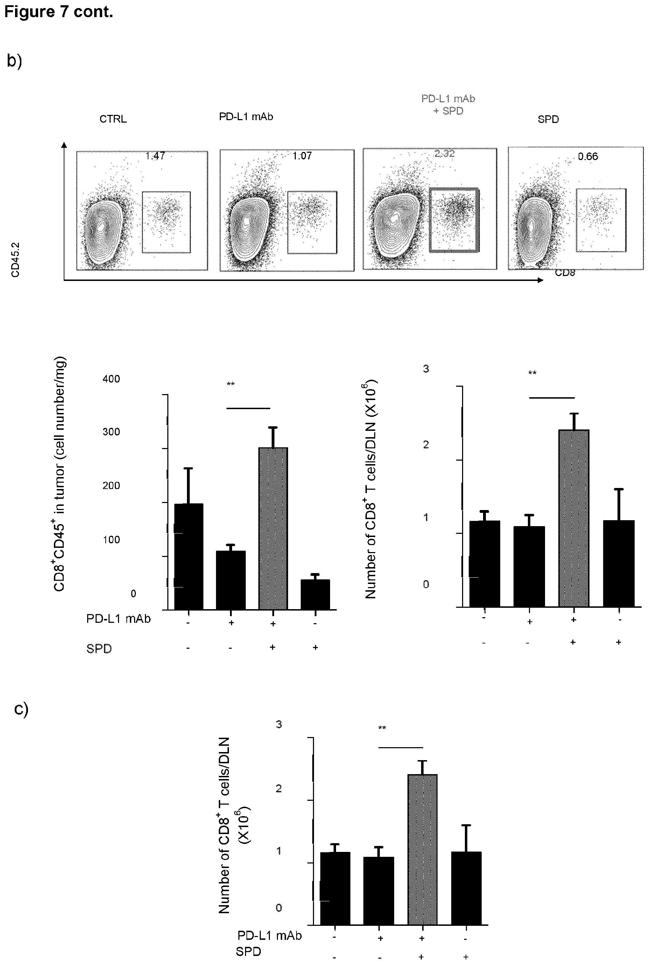

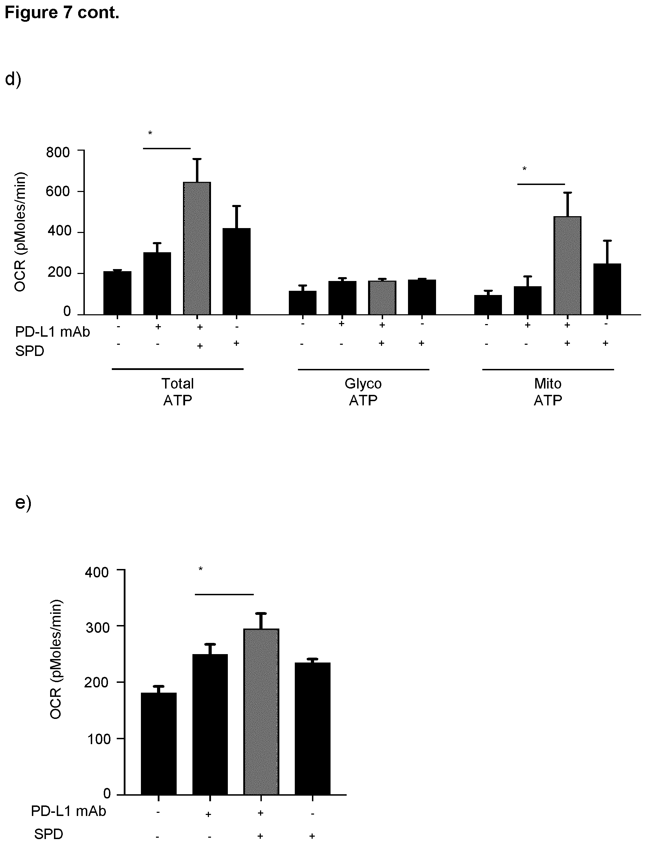

[Fig. 7] MC38 cells (5x10$^5$) were intradermally inoculated into 56-week old mice, which were treated along with the treatment schedule described in Fig. 1. PD-L1 antibody (2 mg/kg) and SPD (2 mg/kg) were used. a) Tumor growth curve and survival curve are shown. b) The frequency of CD8$^+$ T cells among lymphocytes infiltrating into tumor (in CD45$^+$ gate) and the absolute number of CD8$^+$ T cells per mg tumor 13 days after tumor inoculation are shown. c) CD8$^+$ T cells were isolated from spleen cells on the same schedule as b) and cultured for 20 hours under the stimulation with CD3/CD28. Cell proliferation was examined by means of $^3$H uptake. d) CD8$^+$ T cells were isolated from draining lymph nodes on the same schedule as b) and glycolytic pathway-derived or mitochondrial metabolism-derived ATP production was calculated by Seahorse. e) Spare respiratory capacity (SRC) was also calculated in the experiment (d).

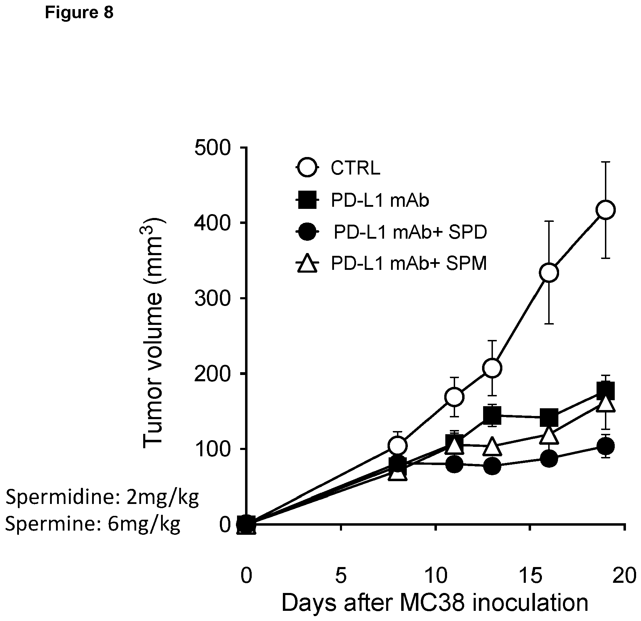

[Fig. 8] MC38 cells (5x10$^5$) were intradermally inoculated into C57BL/6 mice. Ten days after inoculation, PD-L1 antibody (2 mg/kg), PD-L1 antibody + SPD (2 mg/kg) or PD-L1 antibody + SPM (6 mg/kg) was administered. Subsequently, CD-L1 antibody was administered 4 times at a regular interval of 5 days, whereas SPD and SPM were administered 8 times at a regular interval of 3 days. Tumor growth curve is shown.

Description of Embodiments for Carrying out the Invention

Hereinbelow, the present invention will be described more specifically.

The present invention provides a pharmaceutical composition which increases the function of oxidative phosphorylation in T cells.

T cells are lymphocytes that originate from hematopoietic stem cells born in the bone marrow and which differentiated in the thymus gland, where they mature to express self-tolerant T cell receptor (TCR). T cells mainly exist in the lymphoid tissue and blood and express CD45, CD3, TCR, etc. on their surfaces. Upon recognition of non-self antigen-derived peptide presenting cells (e.g., bacterium-infected cells or cancer cells), T cells are capable of being activated and killing such cells. For T cell activation and division, energy is necessary and produced via the extramitochondrial glycolytic pathway or intramitochondrial oxidative phosphorylation.

The pharmaceutical composition of the present invention may suitably be such that it increases the function of oxidative phosphorylation in (i) naive T cells expressing CCR7, CD45RA and CD62L but not expressing CD45RO and CD44 or (ii) activated T cells not expressing CCR7, CD45RA and CD62L but expressing CD45RO and CD44, with both (i) and (ii) existing in the peripheral blood, lymph nodes or tumor tissues.

T cells may be isolated as described below. Briefly, lymphocytes are mixed with CD8-recognizing antibody bound to magnetic beads, and subsequently, CD8$^+$ T cells alone are isolated with a magnet. Alternatively, CD8$^+$ T cells are gated and isolated by a flow cytometer from lymphocytes stained with anti-CD8 antibody.

The function of oxidative phosphorylation in T cells may be measured as described below. Briefly, the oxygen consumption rate (OCR) of T cells is measured with an extracellular flux analyzer (Seahorse). Baseline OCR and the OCR for the case where carbonyl cyanide-4 (trifluoromethoxy) phenylhydrazone (FCCP), or a reagent that is activated to the maximum extent upon addition of a mitochondrial function terminating agent (oligomycin, Antimycine A/Rotenone: A/R), is added are compared and calculated (Divakaruni AS, Paradyse A, Ferrick DA, Murphy AN, Jastroch M. 2014. Analysis and Interpretation of Microplate-Based Oxygen Consumption and pH data. In Methods in Enzymology, Volume 547, Chapter 16, 309-354).

If, in the above-described measurement method, baseline OCR is increased, spare respiratory capacity (SRC) is increased, or mitochondrial ATP production rate (mitoATP) is increased, it may well be concluded that the function of oxidative phosphorylation in T cells has increased.

According to the illustration diagram given below, SRC and mitoATP can be calculated by the following formulas.

$$SRC \text{ (pmo}_1 \text{ } O_2/min) = OCR_{maximal} - OCR_{basal}$$

$$mitoATP \text{ (pmol ATP/min)} = (OCR_{basal} - OCR_{oligo}) \times 5.5$$

**XF Mito Stress Test (Mitochondrial Respiration)**

If used for therapy in combination with a PD-1 signaling inhibitor (e.g., PD-1 inhibitory antibody), the pharmaceutical composition of the present invention is capable of enhancing the effect of the PD-1 signaling inhibitor (e.g., anti-tumor effect, anti-infective effect).

Further, by using it in combination with a PD-1 signaling inhibitor (e.g., PD-1 inhibitory antibody), the pharmaceutical composition of the present invention is capable of producing at least one of the following effects:

- increasing the number of CD8+ T cells in draining lymph nodes
- increasing the ratio of effector T cells (P3) in draining lymph nodes (number of effector T cells relative to number of CD8+ T cells) or the absolute number of P3
- increasing the ratio of CD8+ T cells infiltrating into a lesion site such as tumor (number of CD8+ T cells relative to number of lymphocytes (CD45)) or the number of such CD8+ T cells
- enhancing the expression of Sca-1 in CD8+ T cells in draining lymph nodes
- enhancing the cell proliferation and IFN-γ production of CD8+ T cells in draining lymph nodes as stimulated with CD3
- decreasing the ratio of exhausted CD8+ T cells typified by CD8+ PD-1+ Tim-3+ cells infiltrating into lesion site such as tumor (number of exhausted CD8+ T cells relative to total number of CD8+ T cells)
- increasing the glycolysis pathway (ECAR as an indicator) of CD8+ T cells in draining lymph nodes
- increasing spare respiratory capacity (SRC) which shows the potential of energy production of CD8+ T cells in draining lymph nodes

It is considered that by using the pharmaceutical composition of the present invention in combination with a PD-1 signaling inhibitor, exhausted T cells are decreased and hypermetabolism of T cells is triggered to thereby increase the function of T cells.

By combination therapy using the pharmaceutical composition of the present invention and a PD-1 signaling inhibitor, expression levels of mitochondria-related proteins in CD8+ T cells in draining lymph nodes may be increased or decreased, compared to therapy using the PD-1 signaling inhibitor alone.

The pharmaceutical composition of the present invention may suitably comprise a substance that increases the function of oxidative phosphorylation in T cells as an active ingredient. As a substance that increases the function of oxidative phosphorylation in T cells, a polyamine may be given. Polyamines are straight-chain aliphatic hydrocarbons with two or more amino groups and are ubiquitous in living bodies. Polyamines are present mainly in a cell-bound form. In the blood, they are found in high concentrations in erythrocytes and leukocytes. The mode of action of polyamine is diverse. Polyamines have anti-oxidative effect and anti-inflammatory effect, and it is said that polyamines are associated with long survival or anti-aging. Specific examples of polyamine include, but are not limited to, spermine, spermidine and putrescine. A combination of these polyamines may also be used. As polyamine, spermine, spermidine and putrescine are preferable; spermine and spermidine are more preferable; and spermidine is still more preferable. A polyamine may be in the form of a salt with acid such as hydrochloric acid, methanesulfonic acid, fumaric acid or phosphoric acid. Further, polyamine or a salt thereof may form a hydrate with water, or a solvate with an organic solvent such as methanol or ethanol. Such a salt, hydrate or solvate may be one that is acceptable as a pharmaceutical drug.

Moreover, the present invention provides a pharmaceutical composition which has an effect of increasing the function of oxidative phosphorylation in T cells and is administered before, after or simultaneously with the administration of a PD-1 signaling inhibitor. The pharmaceutical composition of the present invention may comprise a substance that increases the function of oxidative phosphorylation in T cells as an active ingredient. As a substance that increases the function of oxidative phosphorylation in T cells, a polyamine may be given. As regards polyamine, reference should be made to the foregoing description.

As used herein, the term "PD-1 signaling" refers to the signal transduction mechanism which PD-1 bears. As one aspect of this mechanism, PD-1 inhibits T cell activation in collaboration with its ligands PD-L1 and PD-L2. PD-1 (Programmed cell death-1) is a membrane protein expressed in activated T cells and B cells. Its ligands PD-L1 and PD-L2 are expressed in various cells such as antigen-presenting cells (monocytes, dendritic cells, etc.) and cancer cells. PD-1, PD-L1 and PD-L2 work as inhibitory factors which inhibit T cell activation. Certain types of cancer cells and virus-infected cells escape from host immune surveillance by expressing the ligands of PD-1 to thereby inhibit T cell activation.

As PD-1 signaling inhibitors, substances which specifically bind to PD-1, PD-L1 or PD-L2 may be given. Such substances include, but are not limited to, proteins, polypeptides, oligopeptides, nucleic acids (including natural-type and artificial nucleic acids), low molecular weight organic compounds, inorganic compounds, cell extracts, and extracts from animals, plants, soils or the like. These substances may be either natural or synthetic products. Preferable PD-1 signaling inhibitors are antibodies. More preferably, antibodies such as anti-PD-1 antibody, anti-PD-LI antibody and anti-PD-L2 antibody may be given. Any type of antibody may be used as long as it is capable of inhibiting PD-1 signaling. The antibody may be any of polyclonal antibody, monoclonal antibody, chimeric antibody, single chain antibody, humanized antibody or human-type antibody. Methods for preparing such antibodies are known. The antibody may be derived from any organisms such as human, mouse, rat, rabbit, goat or guinea pig. As used herein, the term "antibody" is a concept encompassing antibodies of smaller molecular sizes such as Fab, F(ab)'$_2$, ScFv, Diabody, $V_H$, $V_L$, Sc(Fv)$_2$, Bispecific sc(Fv)$_2$, Minibody, scFv-Fc monomer or scFv-Fc dimer.

When the pharmaceutical composition of the present invention is administered before, after or simultaneously with the administration of a PD-1 signaling inhibitor, it may be used as an anti-cancer agent, an anti-infective agent or a combination thereof.

When a combination of the pharmaceutical composition of the present invention and a PD-1 signaling inhibitor is administered as an anticancer agent, target cancers or tumors includes, but are not limited to, leukemia, lymphoma (e.g., Hodgkin's disease, non-Hodgkin's lymphoma), multiple myeloma, brain tumors, breast cancer, endometrial cancer, cervical cancer, ovarian cancer, esophageal cancer, stomach cancer, appendix cancer, colon cancer, liver cancer, gallbladder cancer, bile duct cancer, pancreatic cancer, adrenal cancer, gastrointestinal stromal tumor, mesothelioma, head and neck cancer (such as laryngeal cancer), oral cancer (such as floor of mouth cancer), gingival cancer, tongue cancer, buccal mucosa cancer, salivary gland cancer, nasal sinus cancer (e.g., maxillary sinus cancer, frontal sinus cancer, ethmoid sinus cancer, sphenoid sinus cancer), thyroid cancer, renal cancer, lung cancer, osteosarcoma, prostate cancer, testicular tumor (testicular cancer), renal cell carcinoma, bladder cancer, rhabdomyosarcoma, skin cancer (e.g., basal cell cancer, squamous cell carcinoma, malignant melanoma, actinic keratosis, Bowen's disease, Paget's disease) and anal cancer.

When a combination of the pharmaceutical composition of the present invention and a PD-1 signaling inhibitor is administered as an anti-infective agent, target infections include, but are not limited to, bacterial infections [various infections caused by Streptococcus (e.g., group A β hemolytic streptococcus, pneumococcus), Staphylococcus aureus (e.g., MSSA, MRSA), Staphylococcus epidermidis, Enterococcus, Listeria, Neisseria meningitis aureus, Neisseria gonorrhoeae, pathogenic Escherichia coli (e.g., 0157:H7), Klebsiella (Klebsiella pneumoniae), Proteus, Bordetella pertussis, Pseudomonas aeruginosa, Serratia, Citrobacter, Acinetobacter, Enterobacter, mycoplasma, Clostridium or the like; tuberculosis, cholera, plague, diphtheria, dysentery, scarlet fever, anthrax, syphilis, tetanus, leprosy, Legionella pneumonia (legionellosis), leptospirosis, Lyme disease, tularemia, Q fever, and the like], rickettsial infections (e.g., epidemic typhus, scrub typhus, Japanese spotted fever), chlamydial infections (e.g., trachoma, genital chlamydial infection, psittacosis), fungal infections (e.g., aspergillosis, candidiasis, cryptococcosis, trichophytosis, histoplasmosis, Pneumocystis pneumonia), parasitic protozoan infections (e.g., amoebic dysentery, malaria, toxoplasmosis, leishmaniasis, cryptosporidiosis), parasitic helminthic infections (e.g., echinococcosis, schistosomiasis japonica, filariasis, ascariasis, diphyllobothriasis latum), and viral infections [e.g., influenza, viral hepatitis, viral meningitis, acquired immune deficiency syndrome (AIDS), adult T-cell leukemia, Ebola hemorrhagic fever, yellow fever, cold syndrome, rabies, cytomegalovirus infection, severe acute respiratory syndrome (SARS), progressive multifocal leukoencephalopathy, chickenpox, herpes zoster, hand-foot-and-mouth disease, dengue, erythema infectiosum, infectious mononucleosis, smallpox, rubella, acute anterior poliomyelitis (polio), measles, pharyngoconjunctival fever (pool fever), Marburg hemorrhagic fever, hantavirus renal hemorrhagic fever, Lassa fever, mumps, West Nile fever, herpangina and chikungunya fever].

When the pharmaceutical composition of the present invention is administered before, after or simultaneously with the administration of a PD-1 signaling inhibitor, it may be used in combination with the PD-1 signaling inhibitor or may be formulated with the PD-1 signaling inhibitor as a single dosage.

When a PD-1 signaling inhibitor and the pharmaceutical composition of the present invention are used in combination, the PD-1 signaling inhibitor and the pharmaceutical composition may be administered separately.

When a PD-1 signaling inhibitor and the pharmaceutical composition of the present invention are formulated as a single dosage, a combination drug containing the PD-1 signaling inhibitor and the pharmaceutical composition of the present invention may be prepared.

The pharmaceutical composition of the present invention is administered to human or animal subjects systemically or locally by an oral or parenteral route.

PD-1 signaling inhibitors (e.g., anti-PD-1 antibody, anti-PD-Ll antibody or anti-PD-L2 antibody) may be dissolved in buffers such as PBS, physiological saline or sterile water, optionally filter- or otherwise sterilized before being administered to human or animal subjects by injection or infusion. To the solution of PD-1 signaling inhibitors, additives such as coloring agents, emulsifiers, suspending agents, surfactants, solubilizers, stabilizers, preservatives, antioxidants, buffers, isotonizing agents and the like may be added. As routes of administration, intravenous, intramuscular, intraperitoneal, subcutaneous or intradermal administration and the like may be selected.

The content of the PD-1 signaling inhibitor (e.g., anti-PD-1 antibody, anti-PD-Ll antibody or anti-PD-L2 antibody) in a preparation varies with the type of the preparation and is usually 1-100% by weight, preferably 50-100% by weight. Such a preparation may be formulated into a unit dosage form.

The dose and the number of times and frequency of administration of PD-1 signaling inhibitor (e.g., anti-PD-1 antibody, anti-PD-Ll antibody or anti-PD-L2 antibody) may vary with the symptoms, age and body weight of the human or animal subject, the method of administration, dosage form and so on. For example, in terms of the amount of the active ingredient, 0.1-100 mg/kg body weight, preferably 1-10 mg/kg body weight, may usually be administered per adult at least once at a frequency that enables confirmation of the desired effect.

The active ingredient of the pharmaceutical composition of the present invention may be contained in a preparation comprising a PD-1 signaling inhibitor. Alternatively, the active ingredient either alone or in admixture with an excipient or carrier may be formulated into tablets, capsules, powders, granules, liquids, syrups, aerosols, suppositories, injections or the like. The excipient or carrier may be of any type that is routinely used in the art and pharmaceutically acceptable, with their type and composition being appropriately changed. As a liquid carrier, for example, water, plant oil or the like may be used. As a solid carrier, saccharides such as lactose, sucrose or glucose, starches such as potato starch or corn starch, cellulose derivatives such as microcrystalline cellulose, and the like may be used. Lubricants such as magnesium stearate, binders such as gelatin or hydroxypropyl cellulose, and disintegrants such as carboxymethyl cellulose, and the like may be added. What is more, antioxidants, coloring agents, flavoring agents, preservatives, and the like may also be added.

The pharmaceutical composition of the present invention may be administered via various routes such as oral, transnasal, rectal, transdermal, subcutaneous, intravenous or intramuscular route.

The content of the active ingredient of the pharmaceutical composition of the present invention varies with the type of the preparation and is usually 1-100% by weight, preferably 50-100% by weight. In the case of a liquid, for example, the content of the pharmaceutical composition of the present invention in the preparation is preferably 1-100% by weight. In the case of a capsule, tablet, granule or powder, the content of the pharmaceutical composition of the present invention in the preparation is usually 10-100% by weight, preferably 50-100% by weight, with the balance being the carrier. The preparation may be formulated into a unit dosage form.

The dose and the number of times and frequency of administration of the pharmaceutical composition of the present invention may vary with the symptoms, age and body weight of the human or animal subject, the method of administration, dosage form and so on. For example, in terms of the amount of the active ingredient, approximately 0.001-1000 mg/kg body weight may usually be administered per adult at least once at a frequency that enables confirmation of the desired effect.

The ratio (in mass) of PD-1 signaling inhibitor (e.g., anti-PD-1 antibody, anti-PD-LI antibody or anti-PD-L2 antibody) to the active ingredient of the pharmaceutical composition of the present invention is suitably y from 1:2 to 1:0.1, preferably from 1:0.1 to 1:0.001.

The present invention also provides a method of treating cancer, infection or a combination thereof, comprising administering to a human or animal subject a pharmaceutically effective amount of a pharmaceutical composition that increases the function of oxidative phosphorylation in T cells before, after or simultaneously with the administration of a PD-1 signaling inhibitor. Further, the present invention provides use of a pharmaceutical composition that increases the function of oxidative phosphorylation in T cells for treating cancer, infection or a combination thereof, wherein the pharmaceutical composition that increases the function of oxidative phosphorylation in T cells is administered before, after or simultaneously with the administration of a PD-1 signaling inhibitor. Still further, the present invention provides use of a pharmaceutical composition that increases the function of oxidative phosphorylation in T cells in a method for treating cancer, infection or a combination thereof, wherein the pharmaceutical composition that increases the function of oxidative phosphorylation in T cells is administered before, after or simultaneously with the administration of a PD-1 signaling inhibitor.

Screening for drugs that enhance PD-1 signaling inhibitory activity has become possible by using an effect of increasing the function of oxidative phosphorylation in T cells as an indicator based on the findings obtained by the present invention. Therefore, the present invention also provides a method of screening for drugs that enhance PD-1 signaling inhibitory activity using an effect of increasing the function of oxidative phosphorylation in T cells as an indicator. As regards T cells, the effect of increasing the function of oxidative phosphorylation in T cells and the method of measuring the effect, reference should be made to the foregoing description.

Examples

Hereinbelow, the present invention will be described in more detail with reference to the following Example.

[Example 1]

Materials and Methods

Mice, Cells and Reagents

All mice were maintained under specific pathogen-free (SPF) conditions at the Institute of Laboratory Animals, Graduate School of Medicine, Kyoto University and used under appropriate protocols. C57BL/6 female mice (5 to 6 wk old) were obtained from Charles River Laboratories Japan (Yokohama). Murine colon adenocarcinoma MC38 cells were kindly provided by Dr. Jim Allison (Memorial Sloan-Kettering Cancer Center, New York, NY). This cell line was cultured in DMEM (Invitrogen) containing 10% heat inactivated FBS and 1% antifungal antibiotic (Invitrogen). Absence of infection with mycobacteria was already confirmed. Spermidine (Nakarai) was dissolved in purified water from a fresh, unused vial at each time of use in the series of experiments. The dissolved reagent was diluted with PBS and inoculated into mice (200 μl/mouse).

Mouse Therapy Model

MC38 cells ($5 \times 10^5$) were intradermally injected on the right flank. Ten days after inoculation, PD-L1 antibody (1-111A; prepared by and stored at the Department of Immunology and Genomic Medicine, Graduate School of Medicine, Kyoto University) (Immunology Letters 84 (2002) 57-62) (2 mg/kg), PD-L1 antibody + SPD (2 mg/kg) or SPD alone was administered to mice. Subsequently, PD-L1 antibody was administered 4 times at a regular interval of 5 days, and SPD was administered 8 times at a regular interval of 3 days. Tumors were measured on every alternate day, and tumor volumes were calculated using the formula for typical ellipsoid, $\pi \times$ (length $\times$ breadth $\times$ height/6).

Enzyme-Linked Immunoassay (ELISA)

IFN-$\gamma$ levels in culture supernatant were measured quantitatively with mouse IFN-$\gamma$ kit (BioLegend) following the instructions on the kit.

Cell Preparation

For analyzing draining lymph nodes, cells from axillary, brachial, and inguinal lymph nodes on the right side of tumor-inoculated mice were harvested and mixed. Averaged cell numbers per one lymph node were used as absolute cell numbers. For tumor analysis, tumor tissues were minced into 2- to 3-mm pieces with scissors and digested with collagenase type IV (Thermo Fisher Scientific) using a gentle MACS Dissociator (Miltenyi Biotec). The numbers of tumor cells per mg were used as absolute numbers.

Flow Cytometry Analysis

The following antibodies recognizing the indicated antigens were used: CD44 (1M7), CD45.2 (104), CD45.1 (A20), CD8 (53-6.7), CD62L (MEL-14) and Sca-1 (D7) from BioLegend. All flow cytometry experiments were performed on FACS Canto II (BD Biosciences) and analyzed using FlowJo software (FLOWJO, LLC). Apoptosis assay was performed with PI (Sigma) and Annexin (BioLegend) following the instructions on the kits.

Measurement of Oxygen Consumption Rates

Oxygen consumption rates were measured using an XF96 Extracellular Flux Analyzer (Seahorse Bioscience). CD8$^+$ T cells ($4 \times 10^5$) per well were seeded in a determined XF96 plate. The four chemical modulators of mitochondrial oxidative phosphorylation, which came with an XF Cell Mito Stress Test Kit (Seahorse Bioscience), were added to the module sequentially. In brief, basal OCR measurement was performed after sequential addition of oligomycin, FCCP, and rotenone/antimycin A. SRC and ATP production rates were calculated by the following formulas:

$$SRC \ (pmo; \ 0_2/min) = OCR_{maximal} - OCR_{basal}$$

$$mitoATP \ (pmol \ ATP/min) = ( \ OCR_{basal} - OCR_{oligo}) \ x \ 5.5.$$

Western Blotting

CD8$^+$ T cells were isolated using mouse CD8 Microbeads (Miltenyi Biotec or Mojo). After washing with PBS twice, $2 \times 10^6$ cells were solubilized in a lysis buffer containing 30 mM Tris-HCl (pH.7.4), 150 mM NaCl, 10% glycerol, 0.1% SDS, 1% Triton-X-100, 0.05% Na-Doc, 5 mM EDTA (pH.8.0), protease inhibitor mixture (Roche Molecular Biochemicals) and phosphatase inhibitors (Nacalai Tesque). After measurement of protein concentrations by DC protein assay (Bio-Rad), 4 $\mu$g of proteins was mounted on 4 to 20% gradient Mini-PROTEAN TGX Gels (Bio-Rad) and electroblotted onto nitrocellulose membranes, which were then incubated in a blocking buffer of TBS containing 1% BSA. Primary antibody incubations were carried out overnight at 4 °C in blocking buffer. After washing, secondary antibody incubations were carried out at room temperature for 40 min in blocking buffer. Blots were developed with enhanced chemiluminescence (Amersham Pharmacia). Primary antibodies recognizing the following proteins were used: Phospho-mTOR (P-mTOR) (Cat#5536), Phospho-AMPKa (P-AMPK) (#2535), 4E-BP1 (#9644), Phospho-Acetyl-CoA Carboxylase (P-ACC) (#3661) and Phospho-PKCa (#9375). All the primary antibodies were obtained from Cell Signaling Technology.

Spermidine Measurement

Quantitative determination of spermidine in medium and cells was performed by liquid chromatograph-tandem mass spectrometry (LC-MS/MS). Spermidine's standard stock solution was diluted with fresh medium, and working solutions of 0.02, 0.05, 0.1, 0.2, and 0.5 $\mu$M were prepared. Then, calibration curves were prepared by the external standard method. Spermidine was extracted from medium by liquid-liquid extraction using water-methanol-chloroform, and extracted from cells by ultrasonication in 80% methanol for 30 minutes. The amount of spermidine in cells was determined by calculating the amount contained in $10^6$ cells from the concentration in extract solution.

Observation of Mitochondria under Electron Microscope

CD8$^+$ T cells ($2\times10^5$) were stimulated with 0.2 $\mu$M spermidine for 1 hour. Then, cells were harvested, embedded in Ipgell (GenoStaff) and fixed with 4% paraformaldehyde and 2% glutaraldehyde. Images were taken with a transmission electron microscope (TEM). Mitochondrial size was determined with ImageJ software. As regards mitochondrial number, average numbers in single cell were determined with Prism software. Blind testing was used for observation and analysis of mitochondria.

Mitochondria-Related Proteomics Analysis

T cells were prepared by two methods. For *in vivo* analysis, 13 days after tumor inoculation and 3 days after the start of therapy, draining lymph nodes from 5 mice were pooled, followed by isolation of CD8$^+$ T cells. From the isolated CD8$^+$ T cells, three samples each consisting of $1\times10^6$ cells were prepared and used for analysis. For *in vitro* experiments, $2\times10^5$ CD8$^+$ T cells stimulated with 0.2 $\mu$M spermidine for 1 hour were used. For mass spectrometry analysis, a Tandem Mass Tag (TMT) system or a Data Independent Acquisition (DIA) system was used. In the analysis of *in vivo* CD8$^+$ T cells (Fig. 3), those proteins which increased by 1.5-fold or more or decreased by 0.6-fold or less were extracted.

Statistical Analysis

Statistical analysis was performed using Prism 6. One-way ANOVA analysis (one-way analysis of variance) followed by multiple comparison test was used to analyze three or more variables. For comparison of two groups, Student t test was used. All statistical analyses were two-sided assuming parametric data. P value of <0.05 was considered significant. The variations of data were evaluated as the means $\pm$ SEM. Five or more samples are believed to be appropriate for the sample size estimate in the current study. Samples and animals were randomly chosen from the pool and treated. No blind testing was used for the treatment of samples and animals.

Results and Discussion

The present inventors have found that spermidine (SPD) is found in high concentrations in CD3-stimulated T cells or culture thereof. Spermine (SPM), another polyamine, was not detected at high levels in CD3-stimulated cells (Fig. 1-1 and Fig. 1-2). When spermidine was used in combination with a PD-1 inhibitory antibody in therapy, the antitumor effect of the PD-1 inhibitory antibody was greatly enhanced (Fig. 1-1b, Fig. 1-2). At the same time, Analysis of the subpopulations of CD8$^+$ T cells in draining lymph nodes (DLNs) were analyzed to reveal an increase not only in the cell number of the draining lymph nodes but also in the frequency and the absolute number of effector T cells (P3) which were important for antitumor immunity (Fig. 1-1c). Moreover, examination of tumor-infiltrating CD8$^+$ T cells revealed that their frequency and number were increased as a result of the combination therapy (Fig. 1-1d). In the combination therapy using SPD, the expression of Sca-1 (an activation marker) was enhanced (Fig. 2-1a); and the cell proliferation and IFN-$\gamma$ production of isolated CD8$^+$ T cells as stimulated with CD3 were also enhanced by the combined use of SPD (Fig. 2-1b). On the other hand, the frequency of exhausted CD8$^+$ T cells typified by CD8$^+$ PD-1$^+$ Tim-3$^+$ cells was decreased significantly (Fig. 2-1c). It is known that oxidative phosphorylation and metabolic pathway of glycolysis are inactivated in exhausted T cells. However, in the combined therapy, the oxygen consumption (OCR: an indicator of oxidative phosphorylation), glycolysis (ECAR: an indicator of glycolysis) and spare respiratory capacity (SRC: showing potential of energy production) of CD8$^+$ T cells were increased as compared to therapy using PD-1 inhibition alone (Fig. 2-1d). These results show that combined use of SPD and PD-1 inhibitory antibody decreases exhausted T cells while enhancing T cell metabolism to thereby increase T cell function.

The effect of SPD for enhancing the antitumor immunity of PD-1 inhibition was also confirmed in BALB/c mouse (with different genetic background) or different cancer species (Fig. 2-2). Even in a cancer (LLC) that is non-responsive to PD-1 inhibitory antibody used alone, SPD allowed the PD-1 inhibitory antibody to exhibit the antitumor effect (Fig. 2-2).

Since enhancement of metabolism occurs due to SPD, CD8$^+$ T cells in draining lymph nodes were isolated during the combined therapy, and quantitative analysis of mitochondria-related protein abundance (proteomics analysis) was performed. The results revealed that levels of 184 proteins out of 203 mitochondria-related proteins changed significantly in combined therapy, compared to PD-1 inhibition alone therapy. Out of the 184 proteins whose level changed, 172 proteins showed increase in expression (Fig. 3a, 3b). Pathway analysis of these proteins revealed that proteins associated with oxidative phosphorylation and metabolic pathways such as glycolysis occupied top positions (Fig. 3c). Further, expression levels of proteins related to electron transport chain which is the place of ATP production were examined individually. As a result, levels of related proteins such as Cytochrome, NADH dehydrogenase and ATPase were remarkably increased in CD8$^+$ T cells under the combined therapy, compared to therapy using PD-1 inhibition alone (Fig. 3d).

In order to investigate the T cell activation mechanism of SPD, mitochondrial activity in CD8$^+$ T cells *in vitro* was examined. CD8$^+$ T cells can be divided by CD44 expression into naive CD8$^+$ T cells (CD44 low) and effector/memory CD8$^+$ T cells (CD44 high). Because of the difference in reactivity to antigen stimulation, these populations were examined separately. The mitochondrial oxygen consumption (OCR) in CD44 low CD8$^+$ T cells was increased by SPD at any time point of 30 minutes, 1 hour, 2 hours and 72 hours after stimulation (Fig. 4a). Based on these data, ATP production was calculated. Both ATP productions via extramitochondrial glycolytic pathway (glyco ATP) and via intramitochondrial pathway (mito ATP) were significantly high 1 hour after stimulation (Fig. 4b). In CD44 low CD8$^+$ T cells, cell proliferation was increased 24 hours after SPD stimulation (Fig. 4c). Since spare respiratory capacity (SRC) which is an indicator of mitochondrial potential and long survival was enhanced 72 hours after cell stimulation (Fig. 4d), viability *in vitro* was confirmed. As a result, apoptotic cells decreased and viable cells increased in the presence of SPD (Fig. 4e). On the other hand, in CD44 high CD8$^+$ T cells, mitochondrial oxygen consumption (OCR) increased 30 minutes after stimulation in the presence of SPD, and decreased 1 hour and 2 hours after stimulation. However, OCR increased again 72 hours after stimulation due to SPD (Fig. 4f). Based on these data, ATP production was calculated. Although ATP production showed a tendency to be enhanced 30 minutes after stimulation, ATP productions via glycolysis (glyco ATP) and via mitochondria (mito ATP) were not enhanced 1 to 2 hours after stimulation (Fig. 4g). Moreover, in CD44 high CD8$^+$ T cells, SPD was not found to increase cell proliferation 24 hours after SPD stimulation (Fig. 4h). However, since SRC (an indicator of mitochondrial potential and long survival) was enhanced 72 hours after stimulation, viability *in vitro* was checked to reveal that apoptotic cells decreased and viable cells increased in the presence of SPD (Fig. 4i, j).

In order to investigate these differences in reactivity to SPD between CD44 low CD8$^+$ T cells and CD44 high CD8$^+$ T cells, mitochondrial morphology was observed under electron microscope before and after SPD stimulation. As a result, in CD44 low CD8$^+$ T cells, SPD stimulation caused no change in mitochondrial size but produced denser structures of cristae (Fig. 5a-c). In CD44 high CD8$^+$ T cells, SPD stimulation caused fusion of mitochondria to give sporadic elongated mitochondria (Fig. 5e). Accordingly, the size per mitochondrion increased and the number of mitochondria per cell decreased (Fig. 5f-g). The cristae were damaged in structures and there were a great number of cells in which no crista were observed with definite structures. Since it is known that mitochondrial fusion is caused by AMPK (energy sensor) signaling or affected by mTOR, phosphorylated (p) AMPK, mTOR activation and phosphorylation and expression of downstream molecules were examined by western blotting. In CD44 low CD8$^+$ T cells, the presence or absence of SPD did not cause much difference in AMPK and mTOR signaling. However, in CD44 high CD8$^+$ T cells, expression levels of pAMPK and its downstream molecules (pACC, pPKCa) were markedly high. These results suggest that, as shown in Fig. 4f-g and Fig. 5e-g, due to SPD stimulation, mitochondrial damage and sharp decrease in ATP occur in a short time in CD44 high CD8$^+$ T cells, causing activation of AMPK. As a result, fusion of mitochondria with good energy production efficiency may have been caused. In Fig. 4e and 4j, SRC and viability 72 hours after SPD stimulation (late phase) were significantly high in both CD44 low CD8$^+$ T cells and CD44 high CD8$^+$ T cells, as compared to control cells. These data suggest that although mitochondrial activity is damaged in a short period in CD44 high CD8$^+$ T cells, it is eventually promoted by an AMPK signaling-mediated recovery system. To verify this possibility, both CD44 low CD8$^+$ T cells and CD44 high CD8$^+$ T cells were harvested 1 hour and 44 hours after SPD stimulation, and subjected to proteomics analysis focusing on mitochondrial proteins. The results revealed that expression levels of mitochondrial proteins and proteins of mitochondria-related metabolism were high in both CD44 low and high CD8$^+$ T cells in the late phase (44 hours after stimulation) (Fig. 6). These results demonstrate that mitochondrial metabolism is promoted by different mechanisms in CD44 low and high CD8$^+$ T cells.

It is known from mouse model experiments that the effect of PD-1 inhibitory antibody therapy decreases in aged mice due to their decreased immunity. In order to examine whether combined use of SPD can restore the cancer immunity decreased by aging, 56-week old mice (Charles River Laboratories Japan (Yokohama)) were subjected to the combination therapy. The details are the same methods as described in paragraph [0044]. As a result, PD-1 inhibitory antibody alone did not show a tumor shrinking effect, whereas the combination therapy exhibited an enhanced tumor shrinking effect (Fig. 7a). The frequency and the absolute number of CD8$^+$ T cells in lymphocytes infiltrating into tumor in a CD45$^+$ gate 13 days after tumor inoculation were examined. As a result, the combination with SPD has been shown to increase the number of CD8$^+$ T cells in lymphocytes infiltrating into tumor (Fig. 7b). CD8$^+$ T cells were isolated from spleen cells 13 days after tumor inoculation and cultured for 20 hours under the stimulation with CD3/CD28. Cell proliferation was examined by means of 3H uptake. As a result, proliferative activation was enhanced in CD8[+] T cells of the SPD combination group (Fig. 7c). CD8[+] T cells were isolated from draining lymph nodes 13 days after tumor inoculation and glycolytic pathway-derived or mitochondrial metabolism-derived ATP production was calculated by Seahorse. Spare respiratory capacity (SRC) was also calculated. As a result, (Fig. 7d) the production of ATP was increased in a mitochondrial metabolism-dependent manner in the SPD combination treatment group and (Fig. 7e) SRCs of CD8[+] T cells were increased in the SPD combination treatment group. Although it has been reported that mitochondrial activity is weakened in senescent cells, the above result suggests that mitochondria in senescent cells are re-activated by SPD to thereby enhance the antitumor effect of PD-1 inhibitory antibody.

SMP, though slightly less effective than SPD, also induced to some extent an enhancement of the antitumor effect of PD-1 inhibitory antibody (Fig. 8).

All publications, patents and patent applications cited herein are incorporated herein by reference in their entirety.

Industrial Applicability

The pharmaceutical composition of the present invention is applicable as an anticancer agent, a therapeutic for infections or a combination thereof if it is combined with a PD-1 signaling inhibitor.

**Claims**

1. A pharmaceutical composition which increases the function of oxidative phosphorylation in T cells.

2. The pharmaceutical composition of claim 1, which comprises a polyamine.

3. The pharmaceutical composition of claim 1 or 2, wherein the polyamine comprises at least one member selected from the group consisting of spermine, spermidine, putrescine, salts thereof and solvates thereof.

4. A pharmaceutical composition which has an action for increasing the function of oxidative phosphorylation in T cells and is administered before, after or simultaneously with the administration of a PD-1 signaling inhibitor.

5. The pharmaceutical composition of claim 4, which comprises a polyamine.

6. The pharmaceutical composition of claim 5, wherein the polyamine comprises at least one member selected from the group consisting of spermine, spermidine, putrescine, salts thereof and solvates thereof.

7. The pharmaceutical composition of any one of claims 4 to 6, wherein the PD-1 signaling inhibitor is an antibody.

8. The pharmaceutical composition of claim 7, wherein the antibody is at least one antibody selected from the group consisting of anti-PD-1 antibody, anti-PD-Ll antibody and anti-PD-L2 antibody.

9. The pharmaceutical composition of any one of claims 4 to 8, which is used as an anti-cancer agent, an anti-infective agent or a combination thereof.

10. The pharmaceutical composition of any one of claims 4 to 9, wherein the PD-1 signaling inhibitor and the pharmaceutical composition are administered separately.

11. The pharmaceutical composition of any one of claims 4 to 9, which is a combination drug comprising the PD-1 signaling inhibitor and the pharmaceutical composition.

12. A method of treating cancer, infection or a combination thereof, comprising administering to a human or animal subject a pharmaceutically effective amount of a pharmaceutical composition which increases the function of oxidative phosphorylation in T cells, wherein the composition is administered to the subject before, after or simultaneously with the administration of a PD-1 signaling inhibitor.

13. Use of a pharmaceutical composition which increases the function of oxidative phosphorylation in T cells for treating cancer, infection or a combination thereof, wherein the composition is administered before, after or simultaneously with the administration of a PD-1 signaling inhibitor.

14. Use of a pharmaceutical composition which increases the function of oxidative phosphorylation in T cells for a method for treating cancer, infection or a combination thereof, wherein the composition is administered before, after or simultaneously with the administration of a PD-1 signaling inhibitor.

## Figure 1-1

a)

b)

## Figure 1-1 cont.

c) Spermidine increases the frequency and number of effector phenotype CD8 T cells in DLN

## Figure 1-1 cont.

d) Spermidine increases CD8[+] T cells infiltrating tumor mass when combined with anti-PD-L1 monoclonal antibodies

## Figure 1-2

**Cellular levels after 48 hours**

Fold change relative to 0 hours

8 — 6 — 4 — 2 — 0

*

n.s.

CD3/CD28    -    +    -    +

**Cellular levels after 96 hours**

Fold change relative to 0 hours

8 — 6 — 4 — 2 — 0

*

n.s.

-    +    -    +

■ Spermidine
☐ spermine

## Figure 2-1

a) Among the activation markers that were detected to show increasing trends with Spermidine combination therapy is Sca-1

| CTRL | PD-L1mAb | PD-L1mAb+ SPD | SPD |
|---|---|---|---|
| 51.8 | 65.3 | 74.7 | 49.1 |

Sca-1

CD8

**SCA-1**

% among CD8⁺ T cells

80 — 60 — 40 — 20 — 0

****

**

| PD-L1 mAb | - | + | + | - |
| Spermidine | - | - | + | + |

**Figure 2-1 cont.**

b)

Proliferation of CD8 — Thymidine incorporation (CPM)

| PD-L1 mAb | - | + | + | - |
| Spermidine | - | - | + | + |

IFNγ in supernatant of CD8 — pg/ml

| PD-L1 mAb | - | + | + | - |
| Spermidine | - | - | + | + |

c) Spermidine deceases the frequency of PD-1+ Tim-3+ (exhausted) CD8 T cell population in TIL

CTRL | PD-L1 mAb | PD-L1 mAb + SPD | SPD

PD-1 / Tim-3

PD-1$^+$ Tim-3$^-$

PD-1$^+$ Tim-3$^+$ — t-test *

PD-1$^-$ Tim-3$^-$

PD-1$^-$ Tim-3$^+$

% among CD45.2$^+$ CD8$^+$

## Figure 2-1 cont.

d)

## Figure 2-2

a) MethA

**Figure 2-2 cont.**

b) CT26

○ Ctrl IgG
▲ anti-PD-L1 mAb
● anti-PD-L1 mAb + SPD#4mg/kg

c) LLC

○ Ctrl IgG
▲ anti-PD-L1
● anti-PD-L1+SPD-4mg

**Figure 2-2 cont.**

d) Rag2 $^{-/-}$

# Figure 3

a)

Protein levels not changed, 19

Differentially expressed proteins, 184

b)

Down-regulated protein, 12

upregulated-regulated protein, 172

c)

Number of proteins

Oxidative phosphorylation

Glycolysis, Purine and glycerolipid metabolism

steroid and fatty acid biosynthesis

Transcription, Translation and Protein export related

MAPK, calcium signaling , insulin, Ras and cGMP-PKG  signaling

## Figure 3 cont.

d) Fold change of ETC protein levels

## Figure 4

a) CD44 low CD8

# Figure 4 cont.

b)

c)

d)

SRC 72 hours CD44 low

e)

CD44 low

## Figure 4 cont.

f) CD44 high CD8

g)

**Figure 4 cont.**

h)

i)

SRC 72 hours

j)

CD44 high

CD44 high

**Figure 5**

a)

CD44 low

b)

**Average size of mitochondria/cell**

c)

**Average number of mitochondria**

e)    CD44 high

**Figure 5 cont.**

f)

g)

i)

**Figure 5 cont.**

j)

# Figure 6

**Figure 6**

Low ■■■ High

| OXPHOS | CD44L 1 hr | CD44H 1 hr | CD44L 44 hrs | CD44H 44 hrs |
|---|---|---|---|---|
| Atp5b | | | | |
| Ndufs1 | | | | |
| Ndufv1 | | | | |
| Ndufa10 | | | | |
| Atp5o; LOC10 | | | | |
| Ndufa9 | | | | |
| Ndufs2 | | | | |
| Ndufv2 | | | | |
| Atp5j | | | | |
| Ndufs3 | | | | |
| Ndufb10 | | | | |
| Ndufa8 | | | | |
| Ndufa5 | | | | |
| Ndufb8 | | | | |
| Ndufb7 | | | | |
| Ndufb9 | | | | |
| Ndufs6 | | | | |
| Ndufs5; BC00 | | | | |
| Atp5k | | | | |
| Ndufa7 | | | | |
| Ndufa6 | | | | |
| Ndufs4 | | | | |
| ND5 | | | | |
| Ndufs8 | | | | |
| Ndufb5 | | | | |
| Ndufs7 | | | | |
| Ndufa11 | | | | |
| Ndufc2; LOC1 | | | | |
| ND4 | | | | |
| Ndufa2 | | | | |
| Ndufa3 | | | | |
| Atp5j2 | | | | |
| Ndufb6 | | | | |
| Atp5l | | | | |
| ND1 | | | | |
| ND3 | | | | |
| ND2 | | | | |
| Ndufb2 | | | | |
| Atp5s | | | | |

## Figure 6 cont.

FAO

| | | | | |
|---|---|---|---|---|
| Hadha | | | | |
| Acaa2 | | | | |
| Acadvl | | | | |
| Gpd2 | | | | |
| Acat1 | | | | |
| Hadhb | | | | |
| Acadl | | | | |
| Acly | | | | |
| Acsl4 | | | | |
| Acads | | | | |
| Acsl5 | | | | |
| Acadm | | | | |
| Cpt1a | | | | |
| Eci1 | | | | |
| Aldh2 | | | | |
| Echs1 | | | | |
| Gyk; Gk | | | | |
| Cpt2 | | | | |
| Mecr | | | | |
| Slc25a20 | | | | |
| Decr1 | | | | |
| Pecr | | | | |
| Acaca | | | | |
| Ech1 | | | | |
| Gcdh | | | | |
| Ehhadh | | | | |

Glycolysis

| | | | | |
|---|---|---|---|---|
| Pkm | | | | |
| Gapdh; Gm20 | | | | |
| Hk1 | | | | |
| Got2 | | | | |
| Aldoa | | | | |
| Pdhb | | | | |
| Ldha | | | | |
| Hk2 | | | | |
| Dlat | | | | |
| Pdhx | | | | |
| Mpc2 | | | | |
| Got1 | | | | |

## Figure 6 cont.

# Figure 6 cont.

| Mito-ribosome | | | | | |
|---|---|---|---|---|---|
| Mto1 | | | | | |
| Mrpl37 | | | | | |
| Mrpl44 | | | | | |
| Mrpl3 | | | | | |
| Mrpl2 | | | | | |
| Mrpl43 | | | | | |
| Dap3 | | | | | |
| Vars2 | | | | | |
| Dnaja3 | | | | | |
| Gtpbp3 | | | | | |
| Mrps22 | | | | | |
| Mrps7 | | | | | |
| Mrps6 | | | | | |
| Mrps16 | | | | | |
| Trmt10c | | | | | |
| Tufm | | | | | |
| Fars2 | | | | | |
| Tsfm | | | | | |
| Mars2 | | | | | |
| Cct7 | | | | | |
| Trmt1 | | | | | |

| PPAR signaling | | | | | |
|---|---|---|---|---|---|
| Acaa1a | | | | | |
| Acadl | | | | | |
| Acsl4 | | | | | |
| Acsl5 | | | | | |
| Acadm | | | | | |
| Cpt1a | | | | | |
| Gyk; Gk | | | | | |
| Cpt2 | | | | | |
| Acox3 | | | | | |
| Pck2 | | | | | |
| Ehhadh | | | | | |
| Dbi | | | | | |
| Acox1 | | | | | |

**Figure 6 cont.**

**Figure 7**

a)

○ CTRL     ● PD-L1 mAb +

● PD-L1 mAb     ▲ SPD

**Figure 7 cont.**

b)

c)

**Figure 7 cont.**

d)

e)

Figure 8

CTRL
PD-L1 mAb
PD-L1 mAb+ SPD
PD-L1 mAb+ SPM

Tumor volume (mm³)

Spermidine: 2mg/kg
Spermine: 6mg/kg

Days after MC38 inoculation

## INTERNATIONAL SEARCH REPORT

| International application No. |
| --- |
| PCT/JP2020/041190 |

### A. CLASSIFICATION OF SUBJECT MATTER

A61K 39/395(2006.01)i; A61K 45/00(2006.01)i; A61K 45/06(2006.01)i; A61P
31/00(2006.01)i; A61P 35/00(2006.01)i; A61P 43/00(2006.01)i; A61K
31/132(2006.01)i
FI:    A61K45/00; A61P43/00 111; A61P43/00 105; A61K31/132; A61P43/00
        121; A61K45/06; A61K39/395 D; A61K39/395 N; A61P35/00; A61P31/00

According to International Patent Classification (IPC) or to both national classification and IPC

### B. FIELDS SEARCHED

Minimum documentation searched (classification system followed by classification symbols)
A61K39/395; A61K45/00; A61K45/06; A61P31/00; A61P35/00; A61P43/00;
A61K31/132

Documentation searched other than minimum documentation to the extent that such documents are included in the fields searched
    Published examined utility model applications of Japan    1922–1996
    Published unexamined utility model applications of Japan    1971–2020
    Registered utility model specifications of Japan    1996–2020
    Published registered utility model applications of Japan    1994–2020

Electronic data base consulted during the international search (name of data base and, where practicable, search terms used)
    JSTPlus/JMEDPlus/JST7580 (JDreamIII);
    CAplus/REGISTRY/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| X | CN 106399380 A (SHANGHAI YUNSHUN BIOTECHNOLOGY CO., LTD.) 15 February 2017 (2017-02-15) in particular, abstract, claims, example 6, fig. 6 | 1–14 |
| X | CN 106434754 A (SHANGHAI YUNSHUN BIOTECHNOLOGY CO., LTD.) 22 February 2017 (2017-02-22) in particular, abstract, claims, examples 6, 7 | 1–14 |
| X | JP 2014-529620 A (NUTRIALYS MEDICAL NUTRITION SA) 13 November 2014 (2014-11-13) in particular, claims, etc. | 1–3 |
| P, A | FAN, J. et al., "Spermidine as a target for cancer therapy.", Pharmacol Res., May 2020, vol. 159, pp. 1-11 (no. 104943) abstract, fig. 2 | 1–14 |

☐ Further documents are listed in the continuation of Box C.    ☒ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 22 December 2020 (22.12.2020) | 28 December 2020 (28.12.2020) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

Form PCT/ISA/210 (patent family annex) (January 2015)

| INTERNATIONAL SEARCH REPORT | | International application No. | |
|---|---|---|---|
| Information on patent family members | | PCT/JP2020/041190 | |

| Patent Documents referred in the Report | Publication Date | Patent Family | Publication Date |
|---|---|---|---|
| CN 106399380 A | 15 Feb. 2017 | (Family: none) | |
| CN 106434754 A | 22 Feb. 2017 | (Family: none) | |
| JP 2014-529620 A | 13 Nov. 2014 | WO 2013/030512 A1 claims US 2014/0294799 A1 EP 2750710 A1 FR 2979241 A1 CA 2847001 A1 | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019205550 A

**Non-patent literature cited in the description**

- **BRAMER J ; RECKAMP K et al.** Nivolumab versus Docetaxel in Advanced Nonsquamous Non-Small-Cell Lung Cancer. *N Engl J Med,* 2015, vol. 373, 1627-1639
- **HAMANISHI J ; MANDAI M ; IKEDA T et al.** Safety and Antitumor Activity of Anti-PD-1 Antibody, Nivolumab, in Patients with Platinum-Resistant Ovarian Cancer. *J Clin Oncol,* 2015, vol. 33, 4015-4022
- **MOTZER RJ ; ESCUDIER B ; MCDERMOTT DF et al.** Nivolumab versus Everolimus in Advanced Renal-Cell Carcinoma. *N Engl J Med,* 2015, vol. 373, 1803-1813
- Analysis and Interpretation of Microplate-Based Oxygen Consumption and pH data. **DIVAKARUNI AS ; PARADYSE A ; FERRICK DA ; MURPHY AN ; JASTROCH M.** Methods in Enzymology. 2014, vol. 547, 309-354
- *Immunology Letters,* 2002, vol. 84, 57-62